(19) Europäisches Patentamt
European Patent Office
Office européen des brevets

(11) Publication number: **0 108 994**
**A1**

# (12) EUROPEAN PATENT APPLICATION

(21) Application number: **83110884.0**

(22) Date of filing: **01.11.83**

(51) Int. Cl.³: **C 07 D 417/14**, C 07 D 403/14, C 07 D 417/12, C 07 D 205/08, A 61 K 31/505, A 61 K 31/44, A 61 K 31/425, A 61 K 31/395 // C07D403/12, C07D401/12

(30) Priority: **02.11.82 JP 193239/82**

(43) Date of publication of application: **23.05.84 Bulletin 84/21**

(84) Designated Contracting States: **BE CH DE FR GB IT LI NL**

(71) Applicant: **KANEGAFUCHI KAGAKU KOGYO KABUSHIKI KAISHA, 2-4 Nakanoshima 3-chome, Kita-ku Osaka-shi (JP)**

(72) Inventor: **Shimazaki, Masami, 8-4, Nishihata 3-chome, Takasago-shi Hyogo-ken (JP)**
Inventor: **Ideguchi, Satoshi, 7-20, Miyamadai 3-chome Tarumi-ku, Kobe-shi Hyogo-ken (JP)**
Inventor: **Murakami, Hiroshi, 2-63, Okihama-cho Takasago-cho, Takasago-shi Hyogo-ken (JP)**
Inventor: **Nagashima, Nobuo, 2-63, Okihama-cho Takasago-cho, Takasago-shi Hyogo-ken (JP)**
Inventor: **Ueyama, Noboru, 2-63, Okihama-cho Takasago-cho, Takasago-shi Hyogo-ken (JP)**
Inventor: **Kinoshita, Koichi, 2-63, Okihama-cho Takasago-cho, Takasago-shi Hyogo-ken (JP)**
Inventor: **Ohashi, Takehisa, 9-403, 4-1, Tsurukabuto Nada-ku, Kobe-shi Hyogo-ken (JP)**
Inventor: **Watanabe, Kiyoshi, 15-41, Matsugaoka 5-chome, Akashi-shi Hyogo-ken (JP)**
Inventor: **Ohno, Masaji, 1565-13, Koshigoe, Kamakura-shi Kanagawa-ken (JP)**

(74) Representative: **Türk, Dietmar, Dr. rer. nat. et al, Redies, Redies, Türk & Gille Patentanwälte Brucknerstrasse 20, D-4000 Düsseldorf 13 (DE)**

## (54) Process for preparation of optically active (4R)-substituted monocyclic beta-lactam compounds.

(57) An improved process for preparing an optically active (4R)-substituted monocyclic β-lactam compound by subjecting a starting optically active compound to reaction comprising the steps of

(a) conversion of the azido group at C–3 position into amino group with a reducing reagent;

(b) acylation of the amino group;

(c) substitution of the $R^1$ group at C–4 position for a nucleophile and

(d) sulfonation of the N–1 position at a temperature of about 20 to 70 °C.

The products of the present invention are useful as antibiotics and powerful β-lactamase inhibitors.

EP 0 108 994 A1

ACTORUM AG

PROCESS FOR PREPARATION OF OPTICALLY ACTIVE (4R)-SUBSTITUTED MONOCYCLIC β-LACTAM COMPOUND

The present invention relates to a novel process for the preparation of an optically active (4R)-substituted monocyclic β-lactam compound. More particularly, the present invention relates to a process for preparing an optically active (4R)-substituted monocyclic β-lactam compound represented by the formula (I):

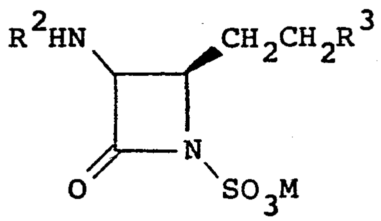

(I)

wherein $R^2$ is an acyl group; $R^3$ is hydrogen atom, hydroxyl, an alkoxy, azido, amino, formimidoylamino, cyano, thiocyanato, mercapto, a substituted thio, dithiocarbamoyl nitro group, or a halogen atom; M is hydrogen atom or a cation, which comprises subjecting a starting optically active compound represented by the formula (II):

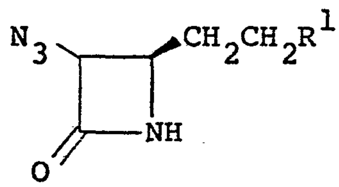

(II)

wherein $R^1$ is alkylsulfonyloxy, aralkylsulfonyloxy, arysulfonyloxy group or a halogen atom,
to reaction comprising the steps of

(a) conversion of the azido group at C-3 position into amino group with a reducing reagent;

(b) acylation of the amino group;

(c) substitution of the $R^1$ group at C-4 position for a nucleophile and

(d) sulfonation of the N-1 position at a temperature of about 20°C to about 70°C.

The products of the present invention are useful as antibiotics and powerful β-lactamase inhibitors. There are continuing needs for new antibiotics because a continued wide scale use selectively gives rise to resistant strains of pathogens. In addition, the known antibiotics suffer from the disadvantage of being effective only against certain types of microorganisms.

Since the monocyclic β-lactam compounds having a sulfo group at N-1 position and a substituent at C-4 position were found in nature [e.g. Imada et al, Nature, 289, p590-591 (1981); R. B. Sykes et al, Nature, 291, P489-491 (1981)], there have been known processes for preparing the derivatives thereof (cf. Japanese Unexamined Patent Publication Nos. 125362/1981 and 131758/1982). In the former publication, though there is disclosed a synthetic process of (3S)-amino-(4R)-methylazetidin-2-one from allothreonine as a starting material, it is difficult to prepare compounds having a substituent other than methyl group at (4R)-position in this method. In the latter publication, a starting material of the (4R)-substituted monocyclic β-lactam compound having an organic residue bonding to C-4 position through two carbon atoms is prepared according to the method for the preparation of cis-configuration β-lactam compounds [J. Am. Chem. Soc., 99, p2352 (1977)] using mainly stereoselective [2+2] addition ring-closure reaction by W. F. Hoffman et al. The method, however, gives only a mixture of (3S,4R)-configuration and (3S,4S)-configuration compound in a proportion of 1 : 1.

On the other hand, there is disclosed a process for preparing a 4-methoxycarbonylethyl derivative from 8-oxo-2,2-dimethyl-3-oxa-1-azabicyclo-[4.2.0]octane [disclosed in J. Am. Chem. Soc., 100, p313 (1978)] by Johnes oxidation of the azido group at C-7

position and methyl esterification. In this case, there is no description of the next steps for preparing further various derivatives from the above compounds. Therefore, it is difficult to obtain a (4R)-substituted monocyclic β-lactam compound in pure form.

It is known that a bioactivity of β-lactam antibiotics depends on its stereo chemistry, and the β-lactam antibiotics having (4R)-configuration exhibit particularly powerful bioactivity. For the purpose of preparing an optically active β-lactam compound having (4R)-configuration in pure form never obtained in the conventional methods, the present inventors have done intensive studies to obtain the desired compound from a pure starting compound obtained by enzymatic preparation using microorganisms and then by chemical preparation. In the course of the above studies, there have already been accomplished a process for preparing optically active aminoglutaric acid monocarbonate (Japanese Patent Application No. 132891/1982), a process for preparing an optically active azetidin-2-one derivative from the above monocarbonate by the ring-closure reaction (Japanese Patent Application No. 25380/1982), and a chemical conversion method of C-4 position of the resulting optically active azetidin-2-one derivative (Japanese Patent Application Nos. 49526/1982, 49527/1982, 49528/1982 and 74377/1982).

There hitherto have been some reports on the chemical preparation of monocyclic β-lactam compound [e.g. C.M. Cimarusti, et al, J. Org. Chem. 47, p176, p180 (1982); M. Ochiai et al, Chem. Pharm. Bull., 31, p1874, p2200 (1983)], but these operations can only give a limited kind of optically active 4-substituted β-lactam compound and cannot produce various optically active (4R)-substituted monocyclic β-lactam compounds.

It is an object of the present invention to provide an improved chemical process for the preparation of optically active (4R)-substituted monocyclic β-lactam antibiotics which are useful in both animal and human

therapy.

In accordance with the present invention, there can be provided a process for the preparation of an optically active (4R)-substituted monocyclic β-lactam compound represented by the formula (I):

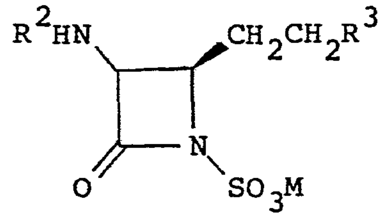

(I)

wherein $R^2$ is an acyl group; $R^3$ is hydrogen atom, hydroxyl, an alkoxy, azido, amino, formimidoylamino, cyano, thiocyanato, mercapto, a substituted thio, dithiocarbamoyl, nitro group or halogen atom; M is hydrogen atom or a cation, which comprises subjecting a starting optically active compound represented by the formula (II):

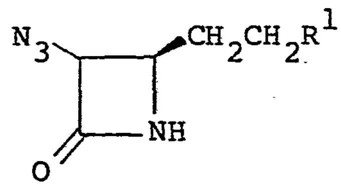

(II)

wherein $R^1$ is alkylsulfonyloxy, aralkylsulfonyloxy, arylsulfonyloxy group or a halogen atom, to reaction comprising the steps of

(a) conversion of the azido group at C-3 position into amino group with a reducing reagent;

(b) acylation of the amino group;

(c) substitution of the $R^1$ group at C-4 position for a nucleophile and

(d) sulfonation of the N-1 position at a temperature of about 20°C to about 70°C.

The starting compound (II) of the present invention is easily derived from (4S)-2-methoxycarbonylmethyl-2-azetidinone, available by chemico-enzymatic reaction [M. Ohno, et al, J. Am. Chem. Soc., 103, p2405 (1981)], for example, by using a method disclosed in

Japanese Patent Application No. 74377/1982. That is, the compound (II) may be prepared by substituion of sulfonate group of the above disclosed compound for a halogen atom in case that $R^1$ group is a halogen atom, or by substitution of hydroxyl group of the (4R)-hydroxyethyl derivative disclosed in the above Application for a halogen atom directly.

According to the process of the present invention, the starting optically active compound (II) could be modified on three positions, that is, on N-1, 3-azido and C-4 positions. Depending on the kinds of reactants, the physicochemical properties of the products, or the reactivity or stability of each functional group, any suitable order of modification may be selected in each case. Representative examples of the process of the present invention are shown in the following reaction diagrams (A) to (G).

(A)

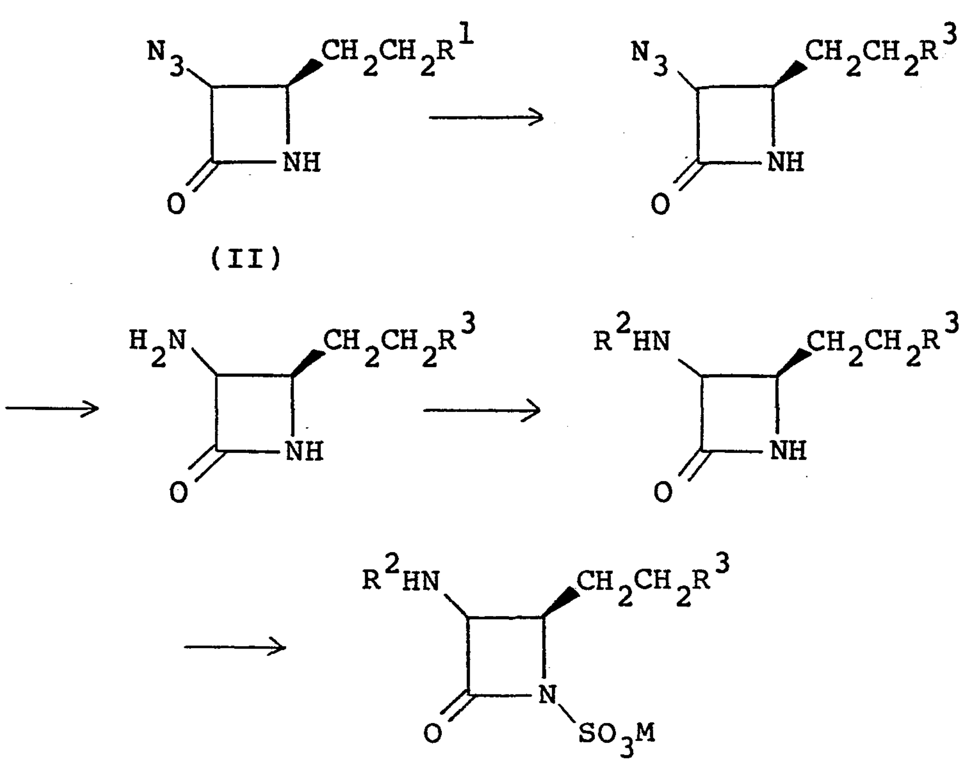

(II)

(I)

(B)

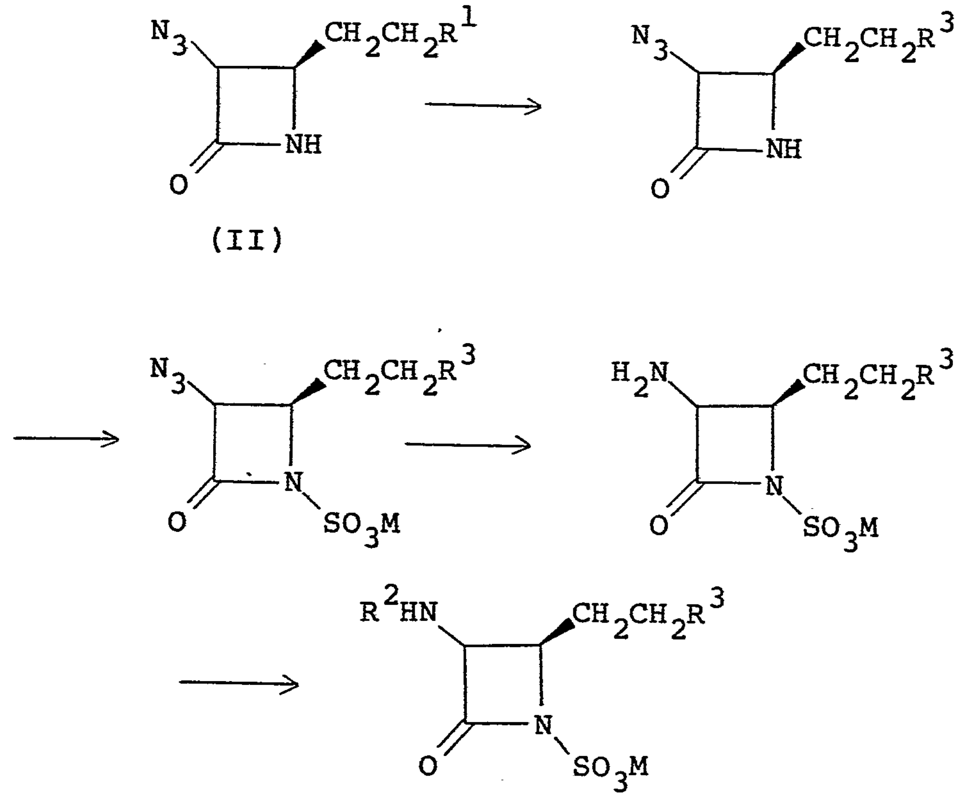

(II)

(I)

(C)

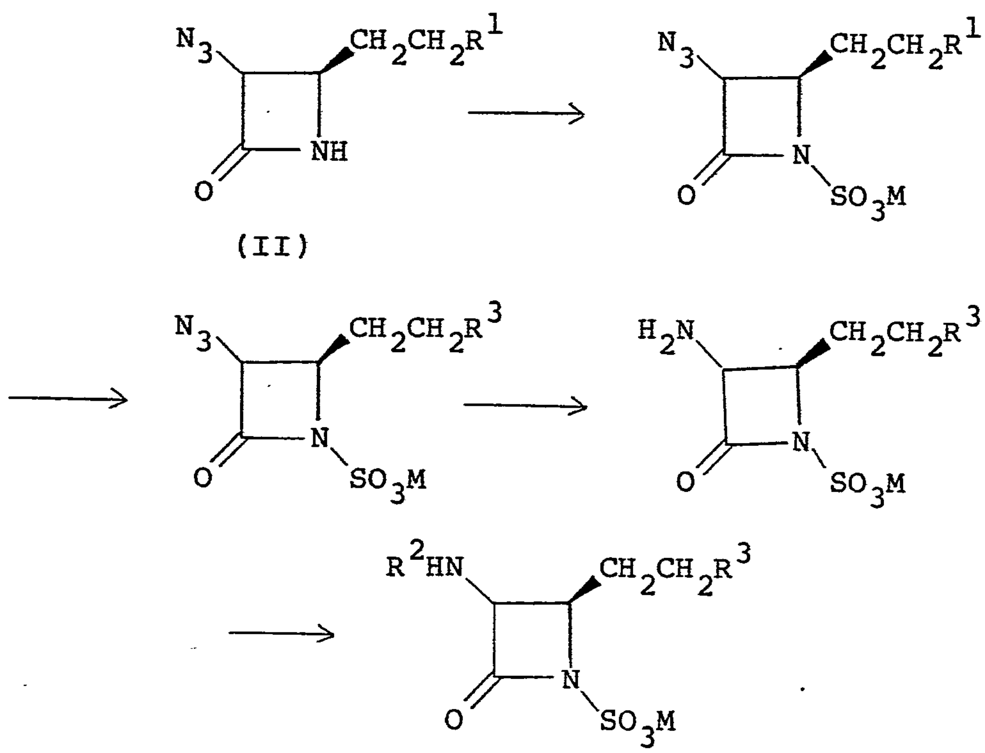

(II)

(I)

(D)

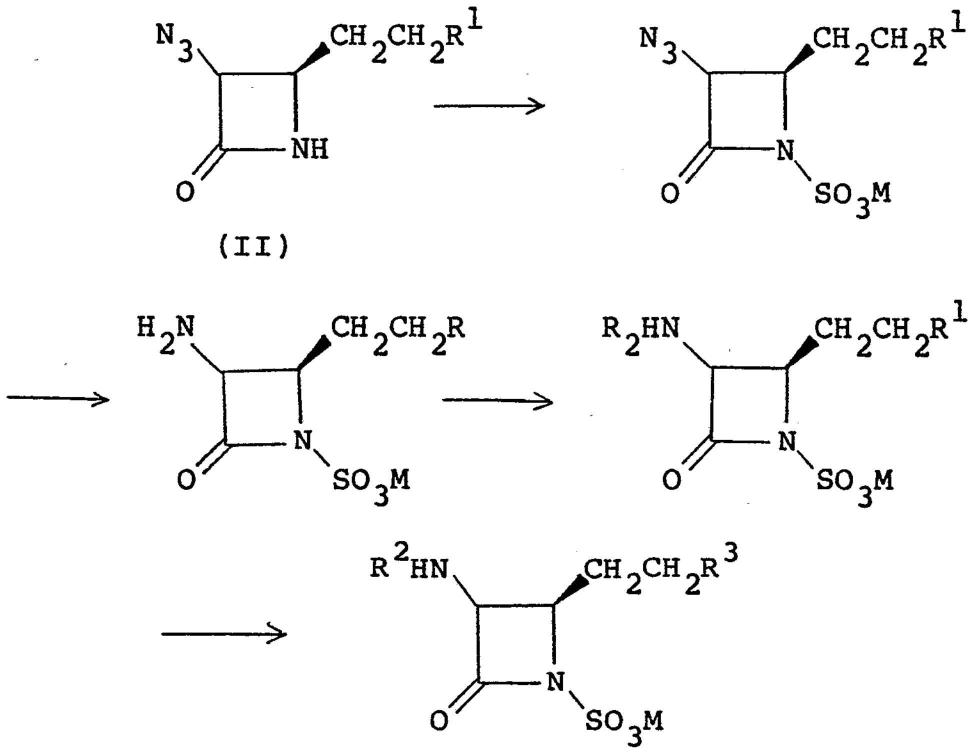

(II)

(I)

(E)

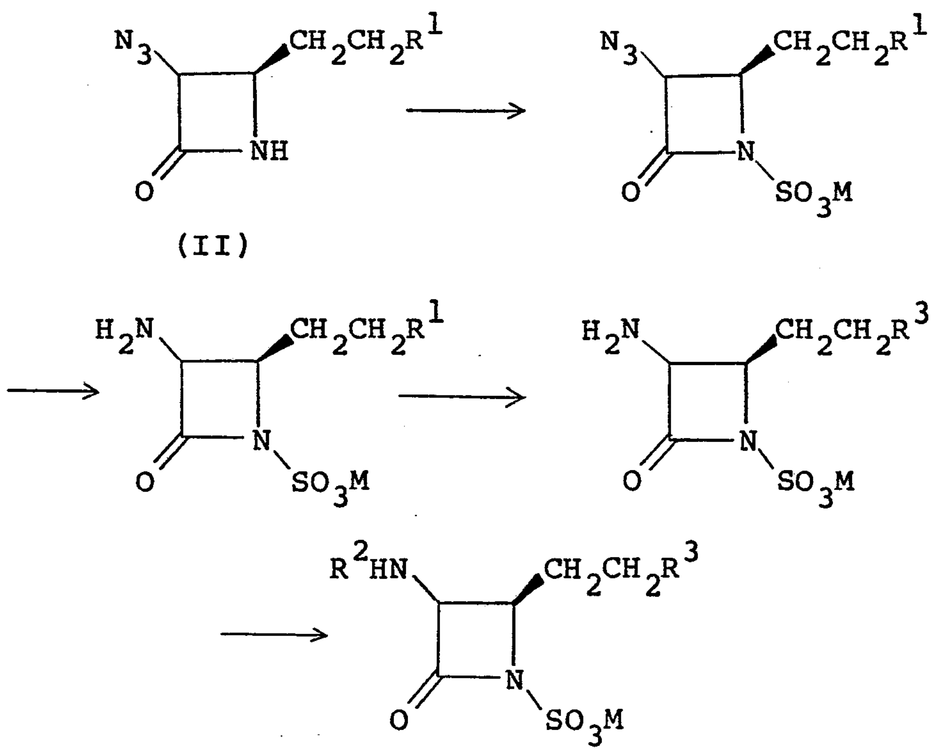

(II)

(I)

(F)

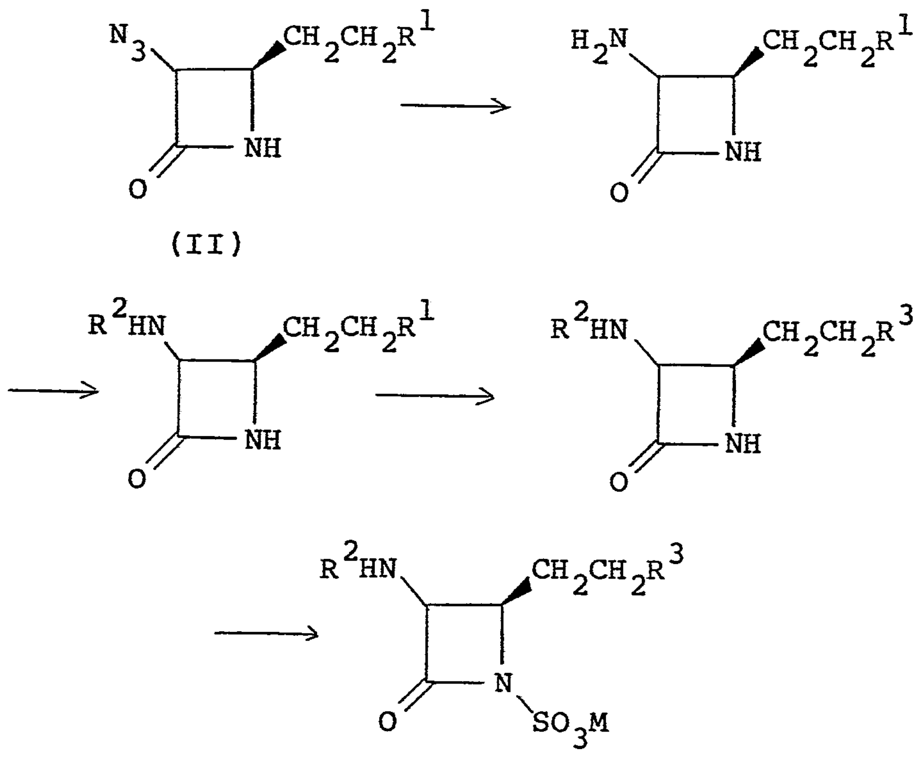

(II)

(I)

(G)

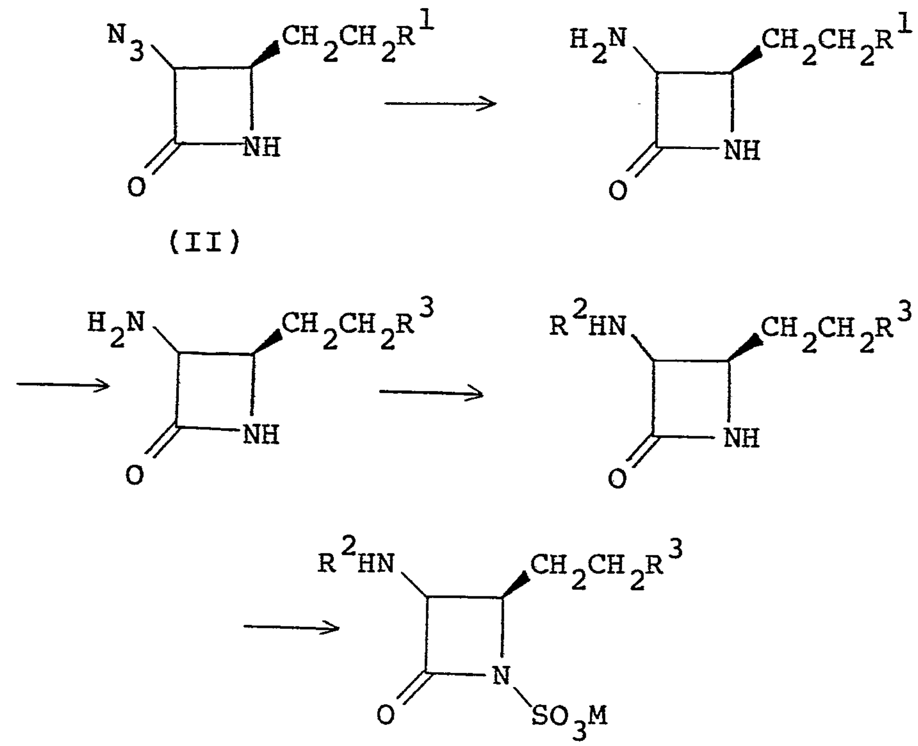

(II)

(I)

In the above processes (A) to (G), the processes (A) to (C) are preferred. The detailed reaction processes are shown as follows:

(A)

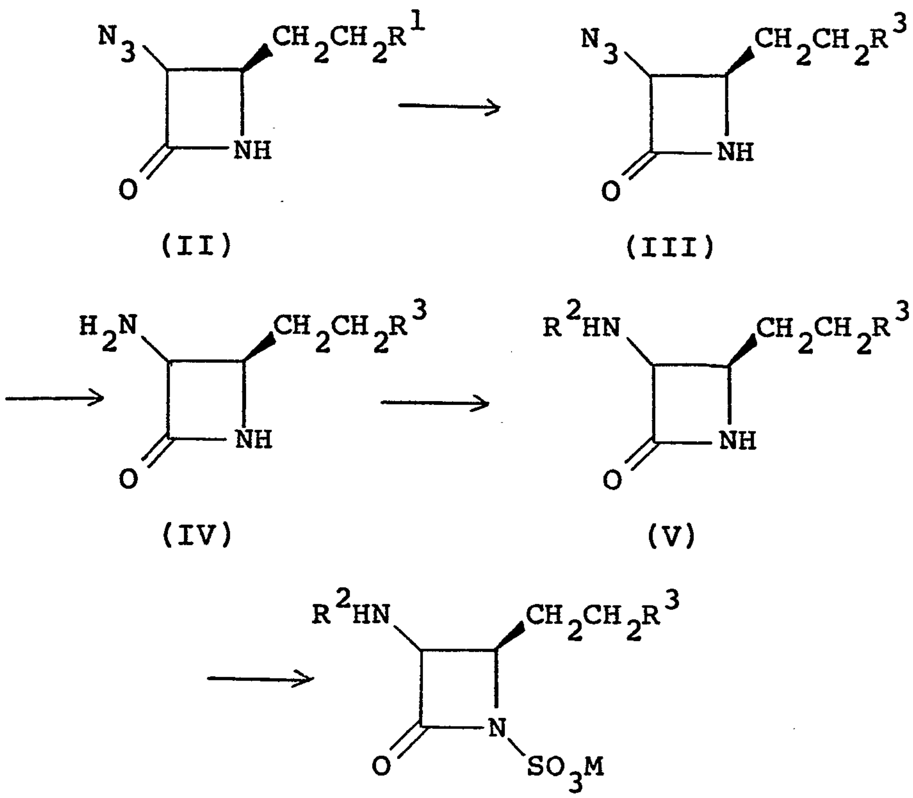

(II) (III) (IV) (V) (I)

The reaction is carried out by

1) reacting the starting optically active compound (II), wherein $R^1$ is as defined above, with a nucleophile to prepare the compound (III), wherein $R^3$ is as defined above;

2) reacting the compound (III) with a reducing reagent to convert the azido group into amino group to produce the compound (IV), wherein $R^3$ is as defined above;

3) acylating the amino group of the compound (IV) to produce the compound (V), wherein $R^2$ and $R^3$ are as defined above;

4) and reacting the compound (V) with sulfonating reagent at about 20°C to about 70°C.

0103994

(B)

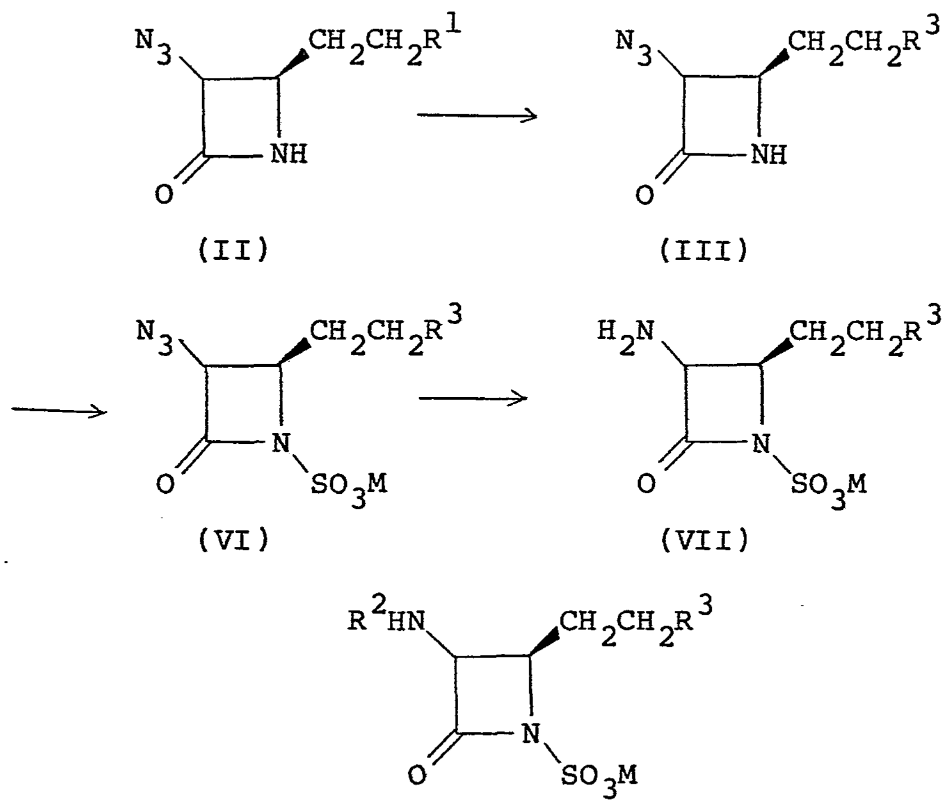

(II) (III)

(VI) (VII)

(I)

The reaction is carried out by

1) reacting the starting optically active compound (II), wherein $R^1$ is as defined above, with a nucleophile to prepare the compound (III), wherein $R^3$ is as defined above;
2) reacting the compound (III) with sulfonating reagent to prepare the compound (VI), wherein $R^3$ and M are as as defined above, at about 20°C to about 70°C.
3) reacting the ocmpound (VI) with a reducing reagent to convert the azido group into amino group to produce the compound (VII), wherein $R^3$ and M are as defined above;
4) and acylating the amino group of the compound (VII).

(C)

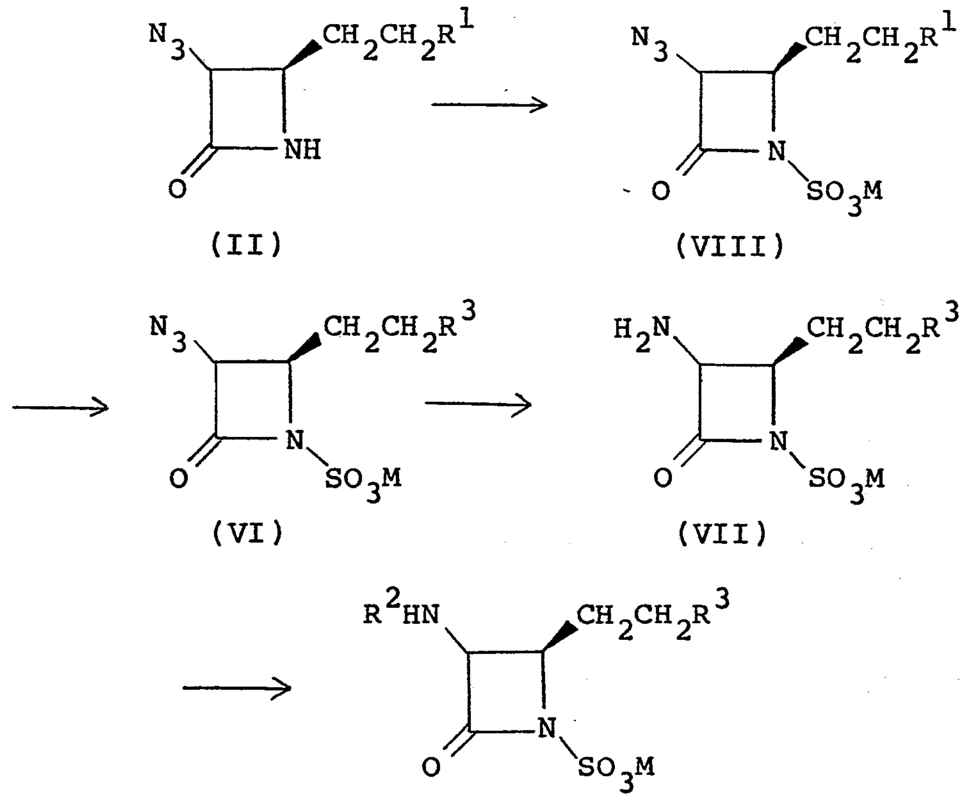

(II) (VIII)

(VI) (VII)

(I)

The reaction is carried out by

1) reacting the starting optically active compound (II), wherein $R^1$ is as defined above, with a sulfonating reagent to prepare the compound (VIII), wherein $R^1$ and M are as defined above;
2) reacting the compound (VIII) with a nucleophile to prepare the compound (VI), wherein $R^3$ and M are as defined above;
3) reacting the compound (VI) with a reducing reagent to convert the azido group into amino group to produce the compound (VII), wherein $R^3$ and M are as defined above;
4) and acylating the amino group of the compound (VII).

According to the process of the present invention, the azido group is converted into amino group by catalytic hydrogenolysis using $PtO_2$, Pd or Ni as a catalyst, or by chemical reduction with zinc powder and acetic acid or with sodium borohydride. Reduction with

hydrogen sulfide or 1,3-propanedithiol is also used. In case of the $R^3$ group being bonded to ethylene group at C-4 position by sulfide-bond, reduction in the presence of 30 % $Pd-BaCO_3$ or Urushibara Nickel as a catalyst is preferably used. Acylation of the resultant amino group is accomplished by conventional peptide forming methods, that is, i) acid chloride method, ii) mixed anhydride method, iii) activated ester method, and iv) DCC method are all used. As the $R^2$ group, all acyl groups which are usually used in penicillin and cephalosporin antibiotics may be used. Examples of $R^2$ group are, for instance, D-phenylglycyl, D-p-hydroxyphenylglycyl, D-p-hydroxyphenyl-N-(4-ethyl-2,3-diketopiperazine-1-carboxamide)glycyl, and the like, and prepferable examples are, for instance, (Z)-2-(2-aminothiazole-4-yl)-2-alkoxyiminoacetyl represented by the formula (IX):

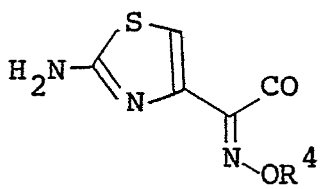

(IX)

wherein $R^4$ is methyl, $CH_2COOR^5$ or $-C(CH_3)_2-COOR^5$ ($R^5$ is hydrogen atom, a cation or an ester group). The cation defined as $R^5$ is an alkali metal such as lithium, sodium or potassium. The ester group defined as $R^5$ is a pharmaceutically acceptable salt such as pivaloyloxymethyl or 1-ethoxycarbonyloxyethyl group.

The $R^3$ group is selected from hydrogen atom, hydroxyl, an alkoxy, azido, amino, formimidoylamino, mercapto, a substituted thio, dithiocarbamoyl, cyano, thiocyanato, nitro group, or a halogen atom. Examples of the alkoxy group are, for instance, methoxy, ethoxy, phenoxy, aryloxy group, and the like. The substituted thio group is represented by the formula SQ, wherein Q is an alkyl, a substituted alkyl, phenyl, aryl, 2-formimidoylethyl, 2-aminoethenyl, 3-amino-2(R)-methylpropyl, 3-amino-2(S)-methylpropyl, 2-amino-2-carboxyethenyl, carbamoylmethyl, or heterocyclic group such as 2-pyrimidyl or 2-pyridyl group. Preferable Q is 2-pyrimidyl or 2-pyridyl group.

The substitution of $R^1$ group at C-4 position with a nucleophilic reagent is carried out in the presence of an organic base such as pyridine, triethylamine, 4-dimethylaminopyridine or diisopropylamine, or an inorganic base such as potassium carbonate. It is preferred to adopt the substitution reaction in the first step of the process. In case of using an alcohol or mercaptane as a nucleophilic reagent, it is preferred to treat the nucleophilic reagent previously with a hydrogenated alkali metal such as sodium hydride or potassium hydride, an organic lithium compound such as butyl lithium, or an inorganic base such as sodium hydroxide, potassium hydroxide, sodium carbonate or potassium carbonate, so as to form an alcoholate or a salt of mercaptide anion. When the $R^3$ group is dithiocarbamate, thiocyanato, cyano or nitro group, an ammonium or an alkali metal salt having such a functional group may be used as a nucleophilic agent in the absence of the above mentioned base. The compound (I), wherein $R^3$ is fluorine atom, is prepared by reacting the compound (II) wherein $R^1$ is mesyloxy or tosyloxy group, with KF in the presence of crown ether such as 18-crown-O-6 in an inert solvent such as diethyl ether, tetrahydrofuran (THF) or 1,2-dimethoxy-ethane (DME).

The $R^1$ group is selected from alkylsulfonyloxy, aralkylsulfonyloxy, arylsulfonyloxy group, or a halogen atom, and preferably mesyloxy, tosyloxyl, or iodine is used.

Introduction of sulfo group to N-1 position is accomplished by using a sulfonating reagent such as a complex of $SO_3$ with an organic base, e.g., pyridine, picoline or N,N-dimethylformamide (DMF). This sulfonation has been reported to be conducted at a room temperature under nitrogen atmosphere taking a long time, but the present inventors have found that the reaction is

more accellerated by heating the reaction mixture at about 20°C to about 70°C, preferably at about 50°C to about 60°C for about 1 to about 5 hours under ordinary atmosphere using DMF as a solvent. The amount of the sulfonating reagent is about 2 to 5 moles per mole of the substrate to be sulfonated, preferably 2 to 4 moles. For example, the sulfonation is completed in 100 % within 3 hours at about 60°C by the process of the present invention, whereas only 50 % or less sulfonation is accomplished at a room temperature even in 48 or more hours by conventional methods. The substituent defined as M is hydrogen atom, a cation or a pharmaceutically acceptable salt, preferably sodium or potassium salt, ammonium salt, or a tetraammonium salt. In case of adopting the process represented by the reaction diagram (B) or (C), tetra-n-butyl-ammonium cation is preferably used as M, for making it easy to extract the intermediate sulfonate with an organic solvent from an aqueous reaction mixture. According to a particularly preferred process for preparing the compound (I), $R^2$ is (Z)-2-[(2-aminothiazol-4-yl)-2-methoxyimino]acetyl, $R^3$ is 2-pyrimidylthio, and M is hydrogen atom, the compound (II), wherein $R^1$ is mesyloxy, tosyloxy or iodine atom, preferably mesyloxy group is first subjected to the substitution of the $R^1$ group for a nucleophile, preferably 2-pyrimidylthio group by reacting the compound (II) with an excess of 2-mercaptopyrimidine in dry DMF in the presence of an organic base, preferably an excess of triethylamine, at room temperatures for 2 to 16 hours to prepare the compound (III). The azido group of the compound (III) is then converted to amino group by treating with hydrogen gas in the presence of Urushibara Nickel A as a catalyst in ethyl alcohol. The product (IV) is acylated directly without purification with (Z)-2-[(2-aminothiazol-4-yl)-2-methoxyimino]acetic acid, preferably using a little excess, by a conventional peptide forming method and preferably by the activated ester method using 1-N-mesyloxybenzotriazole in dry THF

in the presence of an organic base such as triethylamine or by DCC (dicyclohexylcarbodiimide) method in the presence of 1-N-hydroxybenzotriazole monohydrate in dry DMF to produce the compound (V). The introduction of sulfo group to N-1 position of the compound (V) is accomplished by reacting the compound (V) with an excess of $SO_3$-pyridine complex in dry DMF as solvent at about $50^{o}C$ to $60^{o}C$ for about 2 to 5 hours. The optimum amount of the complex is about 2 to 5 moles per mole of the compound (V), preferably 2.5 to 3.5 moles. The sulfonation of N-1 position in the final step of the process is much preferable because it is unnecessary to use tetra-n-butylammonium ion as cation M which is generally utilized to make the intermediate compound such as (VI), (VII) or (VIII) easy to be extracted into organic solvent from aqueous reaction mixture. The product (I) is isolated from diluted reaction mixture using gel absorption chromatograhy.

Though all the processes of the present invention may be carried out in a solvent such as an organic solvent or a mixed solvent of water and an organic solvent, e.g. water-acetone, water-dimethoxyethane, water-THF, or water-dioxane, the solvent used may not be particularly limited.

A bioactivity of β-lactam antibiotics depends on the absolute configuration at C-3 or C-4 position, and generally the (3S) enantiomer exhibits powerful bioactivity in monocyclic β-lactam antibiotics. The present invention includes both (3S, 4R) and (3R, 4R) enantiomer, and a mixture of them is also included.

The present invention is more specifically described and explained by means of the following Examples, wherein Rf values on thin layer chromatogram (TLC) are represented as $R_f^{511} = 0.2$, for example, and the superscript "511" represents a solvent system used. The solvent system is summarized as follows:

511 : EtOAc : EtOH : $H_2O$ = 5 : 1 : 1 (v/v)

311 : EtOAc : EtOH : $H_2O$ = 3 : 1 : 1 (v/v)

411 : n-BuOH : AcOH : $H_2O$ = 4 : 1 : 1 (v/v)

TAxy : toluene : acetone = x : y (v/v)

TExy : toluene : EtOH = x : y (v/v)

The β-lactam compound was detected as a brown spot on silica gel plate by spraying 0.5 % ethanolic solution of Ninhydrin after treatment with spraying 47 % HBr and heating the plate at 150° to 200°C for a few minutes.

## Example 1

Preparation of (Z)-[3S,4R]-3-[2-(2-aminothiazol-4-yl)-2-methoxyimino]acetamido-4-[2-(2-pyrimidylthio)ethyl]-2-azetidinone-1-sulphonic acid (I)

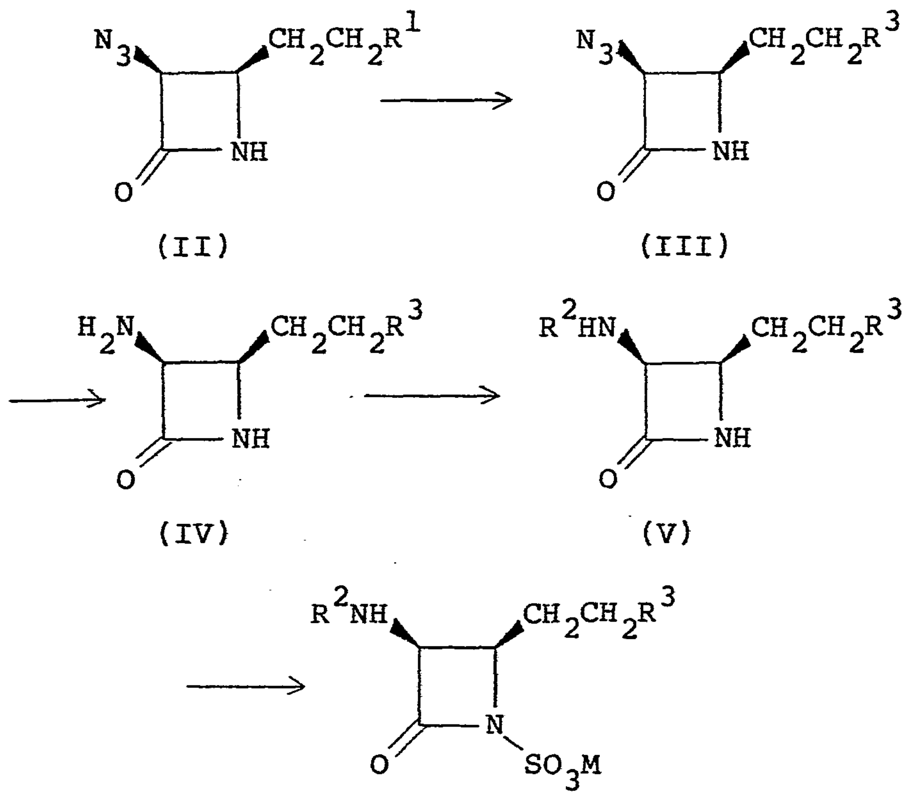

(II) (III) (IV) (V) (I)

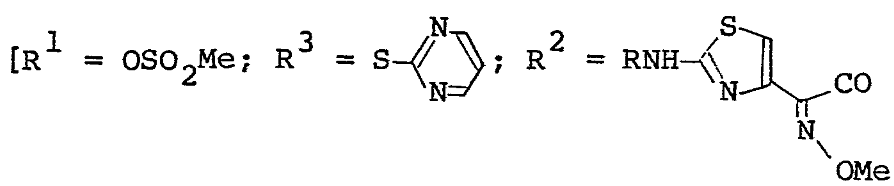

(R = H or $Cl_3CCH_2OC$)]
O

A. Preparation of [3S,4R]-3-azido-4-[2-(2-pyrimidylthio) ethyl]-2-azetidinone (III).

To a mixture of the compound (II) (590 mg, 2.5 mmoles) and 2-mercaptopyrimidine (340 mg, 1.2 mole.eq.) in DMF (5 ml) was added triethylamine (0.5 ml) and the homogeneous reaction mixture was stirred at a room temperature for 16 hours under nitrogen atmosphere. After dilution of the reaction mixture with water (50 ml), the product (III) was extracted with ethyl acetate. The extract was then washed with aqueous $NaHCO_3$ to remove excess 2-mercaptopyrimidine and dried over anhydrous $MgSO_4$. Evaporation of the solvent left a brown oil (650 mg) which was chromatographed on silica gel (Wakogel C-200: supplied from Wako Pure Chem. Ind. Ltd., 20 g) developing with toluene-acetone (2 : 1, v/v) to give the compound (III) (540 mg, 86 %) as yellow syrup.

$R_f^{TAll} = 0.56$

$^1H$ NMR(90 MHz, $CDCl_3$): $\delta$ 2.2(2H,m,$C\underline{H}_2CH_2S$), 3.3(2H, m,$CH_2C\underline{H}_2S$), 3.9(1H,m,$H_4$), 4.72(1H,d-d,J=5.1 Hz, 3.0Hz, $H_3$), 6.95(1H,t,$H_{5'}$), 8.49(2H,d,$H_{4'}$,$H_{6'}$)

B. Preparation of [3S,4R]-3-amino-4-[2-(2-pyrimidylthio)-ethyl]-2-azetidinone (IV)

An ethanolic suspension of Urushibara Nickel A (ca. 5 ml) was added to a solution of the compound (III) (1.0 g, 4.0 mmoles) in EtOH (30 ml) and hydrogen gas was passed into the solution for 3 hours at a room temperature. The time course of the reduction was monitored by thin-layer chromatography (TLC). The reaction mixture was filtered through Hyflo Super-Cel (supplied from Wako Pure Chem. Ind., Ltd.) and the cake was washed with three portions of hot EtOH (20 ml). The filtrate and washings were combined and condensed to crude syrup which was chromatographed on Wakogel C-200 (80 g, under $CHCl_3$) developing with $CHCl_3$ : acetone (3 : 1 - 2 : 1) to give the compound (IV) (448 mg, 50 %) as crystalline solids.

$R_f^{CA31} = 0.2$

$^1H$ NMR(90MHz, $CDCl_3$): δ 2.2(2H,m,$C\underline{H}_2CH_2S$), 3.3(2H,m, $CH_2C\underline{H}_2S$), 3.8(1H,m,$H_4$), 4.32(1H,d-d,J=4.9 and 1.5Hz,$H_3$), 7.01(1H,t,$H_5$ on pyrimidine), 7.1(2H,b, $NH_2$), 8.57(2H,d,J=5.1Hz,$H_4$ and $H_6$ on pyrimidine)

C. Preparation of (Z)-[3S,4R]-3-[2-[2-(2,2,2-trichloroethoxycarbonyl)aminothiazol-4-yl]-2-methoxyimino]-acetamido-4-[2-(2-pyrimidylthio)ethyl]-2-azetidinone (V)

An activated ester was prepared by adding 1-mesyloxybenzotriazole (405 mg, 1.9 mmoles) to a mixture of (Z)-2-[2-(2,2,2-trichloroethoxycarbonyl)aminothiazol-4-yl]-2-methoxyiminoacetic acid (715 mg, 1.9 mmoles) and triethylamine (198 mg, 1.96 mmoles) in dry freshly distilled tetrahydrofuran (THF, 10 ml) and stirring at a room temperature for half an hour. The compound (IV) (387 mg, 1.73 mmoles) in THF (5 ml) was then added to the reaction mixture and stirred at a room temperature with monitoring the time course of the coupling reaction by TLC (solvent system: TA12). After 5 hours' stirring the reaction was quenched by the addition of a phosphate buffer (0.5 M, pH 6, 20 ml). Insoluble organic substances separaged as solids which contained the desired compound. The solid was washed with water and dried, and applied to a flash column chromatography on Wakogel C-200 (40 g, L/D=450 mm/15 mmø, toluene : acetone = 95 : 5 to 2 : 3) to give a yellow syrup of the compound (V, $R=Cl_3CCH_2OCO$) (990 mg, 98.5 %).

$R_f^{TA12} = 0.5$

$^1H$ NMR($CD_3OD$ : DMSO-$d_6$ = 1 : 1): δ (DOH was adjusted to 4.7 ppm) 3.99(3H, s,$OCH_3$), 5.07(2H,s,$Cl_3CCH_2$), 5.42(1H,d,J=4.9Hz,$H_3$), 7.23(1H,t,$H_{5'}$), 7.53(1H,s, $H_{5''}$), 8.69(2H,d,$H_{4'}$,$H_{6'}$)

D. Preparation of the compound (I)

0108994

A mixture of the compound (V) (990 mg, 1.7 mmoles) and $SO_3$-pyridine complex (740 mg, 2.7 eq) in dry DMF (10 ml) was stirred at 50° to 60°C for 4 hours. The mixture was then cooled and diluted with the phosphate buffer (0.5 M, pH 6, 40 ml). The pH of the solution was adjusted to 7.4 with 4 % $NH_4OH$ and neutral organic impurities were removed by extracting with ethyl acetate. The product (I, R=$Cl_3CCH_2OCO$) was isolated from the aqueous solution by extracting with ethyl acetate at pH 1 (adjusted with 6N HCl) and usual work up gave off-white solid (900 mg). The solid was applied to deprotection without further purification with zinc powder (850 mg) in THF-1M $KH_2PO_4$ (60 ml - 20 ml) at a room temperature for 16 hours. The reaction mixture was then filtered through Hyflo Super-Cel and the cake was washed with MeOH. The filtrate and washings were combined and organic solvent was evaporated off. The aqueous residue was saturated with KCl and charged to a column of Diaion HP-20 (120 ml, L/D=540 mm/17 mmø, supplied from Mitsubishi Kasei Chem. Ind. Co., Ltd.) which was washed with deionized water (400 ml) and developed with a linear gradient solvent system (deionized water-30 % aqueous acetone = 300 ml - 300 ml). Each fraction having bioactivity against E. coil was checked on TLC and all fractions containing pure compound (I) (R=H) were combined. After removing of acetone, the aqueous solution was lyophilized to give an amorphous solid of the desired compound (I) (400 mg, 48 %, M=H).

$R_f^{511} = 0.25$, $R_f^{311} = 0.48$

$^1$H NMR(90MHz,$D_2O$): $\delta$ (DOH was adjusted to 4.7 ppm) 2.2-2.4(2H,m,C$\underline{H}_2$$CH_2$S), 3.23(2H,m,$CH_2$C$\underline{H}_2$S), 3.87(3H,s,$OCH_3$), 4.4-4.6(1H,m,H4), 5.42(1H,d, J=5.4Hz,$H_3$), 6.87(1H,s,$H_5$ on thiazole), 7.16(1H, t,$H_5$ on pyrimidine), 8.52(2H,d,$H_4$,$H_6$ on pyrimidine $H_5$)

IR $\nu_{max}^{KBr}$: 3450, 2930, 1760, 1660, 1550, 1390, 1050 $cm^{-1}$

Example 2

Preparation of Potassium (Z)-[3S,4R]-3-[2-(2-aminothiazol-4-yl)-2-methoxyimino]-acetamido-4-[2-(2-pyrimidylthio)-ethyl]-2-azetidinone-1-sulfonate (I)

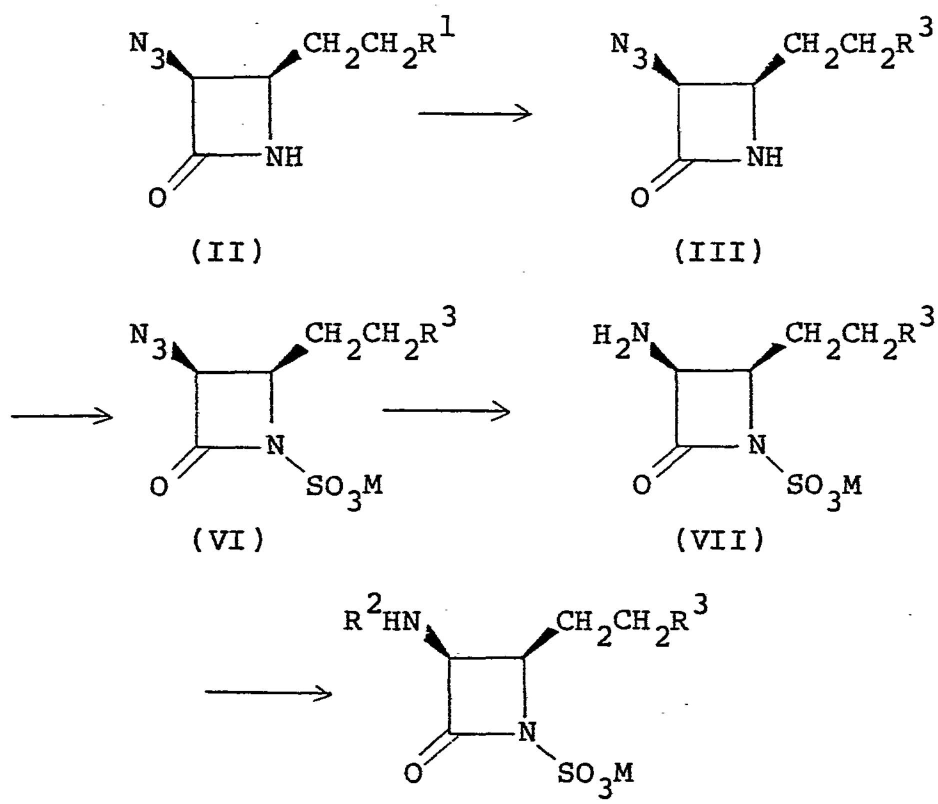

(II) (III) (VI) (VII) (I)

($R^1$ = $OSO_2Me$ or I; $R^3$ = 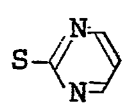; $R^2$ = 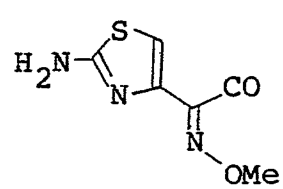)

A. Peparation of [3S,4R]-3-azido-4-[2-(2-pyrimidylthio)-ethyl]-2-azetidinone (III)

A mixture of the compound (II) ($R^1$ = $OSO_2Me$, 230 mg, 0.982 mmole) and NaI (295 mg, 2 eq) in acetone (15 ml) was refluxed for 3 hours. After removal of acetone, water (15 ml) was added and the product was extracted with ethyl acetate (50 ml x 3). The organic

layer was dried over anhydrous $MgSO_4$ and removal of the solvent left a pale yellow solid of the compound (II) ($R^1$ = I, 220 mg, 84 %). The resulting iodide was reacted with 2-mercaptopyrimidine (1.2 eq) in the presence of triethylamine in DMF (5 ml) at a room temperature for 16 hours. The reaction mixture was then worked up in the same manner as in the part A of Example 1 to give the compound (III) (170 mg, 82 %).

B. Preparation of Tetra-n-butylammonium [3S,4R]-3-azido-4-[2-(2-pyrimidylthio)ethyl]-2-azetidinone-1-sulfonate (VI)

The compound (III) (103 mg, 0.413 mmole) and $SO_3$-pyridine complex (2.7 eq) was mixed in dry DMF (2 ml) and warmed at 60°C for 5 hours. DMF was removed by evaporation under reduced pressure and 0.5 M $KH_2PO_4$ (50 ml) was added to the residue and neutral organic impurities were extracted with methylene chloride (25 ml). Tetra-n-butylammonium hydrogen sulfate (140 mg, 0.413 mmole) was added to the aqueous solution and the ammonium salt (VI) was extracted with methylene chloride (50 ml x 3) and dried over anhydrous $MgSO_4$. Removal of the solvent gave a colorless oil of the compound (VI) (M = n-$Bu_4N$, 170 mg, 82 %).

$R_f^{TA12}$ = 0.14

$^1$H NMR(90MHz, $CDCl_3$): $\delta$ 1.0(12H,t,$CH_2CH_2CH_2\underline{CH_3}$), 1.2-1.9(16H,m,$CH_2CH_2CH_2CH_3$), 2.4(2H,m,$\underline{CH_2}CH_2S$), 3.1-3.4(10H,m,$\underline{CH_2}CH_2CH_2CH_3$ and $CH_2\underline{CH_2}S$), 4.25(1H, m, $H_4$), 4.57(1H,d,J=5.4Hz,$H_3$), 6.86(1H,t,$H_{5'}$), 8.43(2H, d,$H_{4'}$ and $H_{6'}$)

C. Preparation of the compound (I)

To a solution of the compound (VI) (210 mg, 0.371 mmole) in EtOH (2 ml) was added Urushibara Nickel A (2 ml of settled volume in EtOH) and stirred at a room temperature under hydrogen atmosphere for 3 hours. The mixture was filtered through Hyflo Super-Cel and the cake was washed with fresh EtOH. The combined filtrate and

washings was condensed to a yellow syrup of crude compound (VII) (M = n-$Bu_4N$) which was then reacted with (Z)-2-(2-aminothiazol-4-yl)-2-methoxyiminoacetic acid (88 mg, 1.17 eq) in the presence of 1-N-hydroxybenzotriazole monohydrate (66 mg. 1.16 eq) and DCC (dicyclohexylcarbodiimide, 112 mg, 1.46 eq) in dry DMF (3 ml) at a room temperature for 20 hours. After removal of DMF under reduced pressure, the residue was coated on Wakogel C-200 (1 g) by using MeOH and subjected to the flash column chromatography (Wakogel C-200, 8g, toluene : acetone = 1 : 1 to 1 : 3) giving a pale yellow solids. The pure compound (I) (M = K, 76 mg, 40 %) was obtained as a pale yellow amorphous powder by treating the crude solids with Dowex 50WX2 ($K^+$, 10 ml, 30 % aq acetone) and Diaion HP-20 (30 ml), and subjecting to lyophilization.

<u>Example 3</u>

Preparation of (Z)-[3R,4R]-3-[2-(2-aminothiazol-4-yl)-2-methoxyimino]acetamido-4-[2-(2-pyrimidylthio)ethyl]-2-azetidinone-1-sulfonic acid (I)

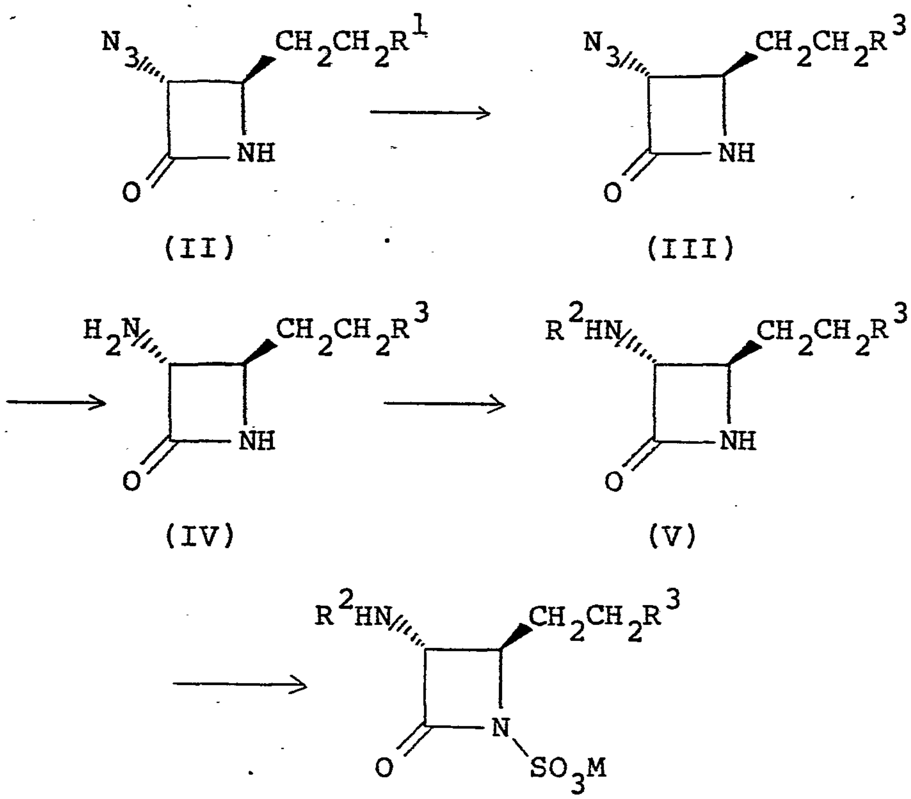

(II) (III)

(IV) (V)

(I)

[$R^1$ = $OSO_2Me$; $R^3$ = S– 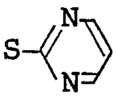 ; $R^2$ = RNH– 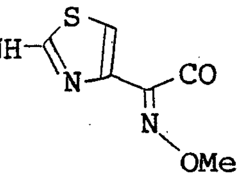

(R = H or $Cl_3CCH_2O\overset{O}{\overset{\|}{C}}$)]

A. Preparation of the compound (III)

By substituting (3R)-enantiomer for (3S)-enantiomer (II) in the procedure of the part A in Example 1, (3R)-enantiomer (III) was obtained (88 %).

$R_f^{511}$ = 0.67, $[\alpha]_D^{25}$ + 74.5° (c 2.0, MeOH)

$^1$H NMR(90MHz, $CDCl_3$): $\delta$ 2.13(2H,q,$C\underline{H}_2CH_2S$), 3.22(2H, m,$CH_2C\underline{H}_2S$), 3.65(1H,t-d,$H_4$), 4.30(1H,d,$H_3$), 6.94(1H,t,$H_{5'}$), 7.26(1H,s,NH), 8.47(2H,d,$H_{4'}$,$H_{6'}$)

IR $\nu_{max}^{neat}$: 3250 (broad), 2105, 1750, 1570, 1550 1380 $cm^{-1}$

B. Preparation of the compound (IV)

By substituting (3R)-enantiomer for (3S)-enantiomer of (III) in the procedure of part the B in Example 1, (3R)-enantiomer of (IV) was obtained as a yellow syrup (68 %).

$R_f^{511}$ = 0.26, $[\alpha]_D^{25}$ + 70.4° (c 1.4, MeOH)

$^1$H NMR(90MHz, $CDCl_3$ : $CD_3OD$ = 3 : 1): $\delta$ 1.97-2.29(2H, m,$C\underline{H}_2CH_2S$), 3.2-3.7(3H,m,$CH_2CH_2S$ and $H_4$), 3.80(1H,d,$H_3$), 7.04(1H,t,$H_{5'}$), 8.55(2H,d,$H_{4'}$ and $H_{6'}$)

IR $\nu_{max}^{neat}$: 3390, 1735, 1545, 1375, 1190 $cm^{-1}$

C. Preparation of the compound (V)

A mixture of the compound (IV) (3R, 68 mg, 0.3 mmole), (Z)-2-[2-(2,2,2-trichloroethoxycarbonyl)amino-

thiazol-4-yl]-2-methoxyiminoacetic acid (113 mg, 0.3 mmole), 1-N-hydroxybenzotriazole monohydrate (44.6 mg, 0.33 mmole) and DCC (66.5 mg, 1.05 eq) in dry DMF (2 ml) was stirred at a room temperature for 16 hours. To the mixture was added diethyl ether (20 ml) and insoluble solids were filtered off which was washed with the ether. The combined filtrate and washings were subjected to the extraction of the product using an additional fresh ether (20 ml). The dried organic solution ($MgSO_4$) was then condensed to a crude solids (361 mg) which was chromatographed on Wakogel C-200 (10 g, toluene : acetone = 9 : 1 to 2 : 1) to give a glassy solid of (V) (152 mg, 87 %).

$R_f^{TA12}$ = 0.6, $[\alpha]_D^{25}$ + 44.1$^o$ (c 1.5, MeOH)

$^1H$ NMR(90MHz, $CDCl_3$): $\delta$ 2.0-2.4(2H,m,$C\underline{H}_2CH_2S$), 3.0-3.5(2H,m,$CH_2C\underline{H}_2S$), 3.90(3H,s,$OCH_3$), 4.87(2H, s,$Cl_3CC\underline{H}_2O$), 4.8-5.1(2H,m,$H_3$ and $H_4$), 6.97(1H,t, $H_5$ of pyrimidine), 7.04(1H,s,$H_5$ of thiazole), 8.54(2H,d,$H_4$ and $H_6$ of pyrimidine)

IR $\nu_{max}^{KBr}$: 3250, 1750 (shoulder), 1740, 1660, 1560, 1550 (sh.), 1380, 1220 $cm^{-1}$

D. Preparation of (Z)-[3R,4R]-3-[2-(2,2,2-trichloroethoxycarbonyl)-aminothiazol-4-yl]-2-methoxyimino]acetamido-4-[2-(2-pyrimidylthio)ethyl]-2-azetidinone-1-sulfonic acid (I)

The complex of $SO_3$ with pyridine (143 mg, 3.5 eq) was added to a solution of (V) (150 mg, 0.257 mmole) in DMF (5 ml) and the mixture was stirred at 55$^o$ to 60$^o$C for 3.5 hours. After cooling, the phosphate buffer (0.2 M, pH 6.8, 20 ml) was added to the mixture and adjusted to pH 1 with 6N $H_2SO_4$. The organic compound was extracted with ethyl acetate (50 ml X 2), washed with aq. NaCl and dried over anhydrous $Na_2SO_4$. Evaporation of the solvent left a brown syrup (200 mg) which was chromatographed on Wakogel C-200) (10 g, EtOAc : EtOH = 9 : 1 to 4 : 1) to give the compound (I, R = $Cl_3CCH_2OCO$) (157 mg, 92 %) as white powder.

$R_f^{511}$ = 0.39

$^1H$ NMR(90MHz, $CD_3OD$): δ 2.0-2.8(2H,m,$C\underline{H}_2CH_2S$), 3.3(2H,m,$CH_2C\underline{H}_2S$), 3.97(3H,s,$OCH_3$), 4.17(1H,m, $H_4$), 4.85(1H,d,J=2.9Hz,$H_3$), 4.93(2H,s,$Cl_3CCH_2O$), 7.07(1H,t,$H_5$ of pyrimidine), 7.35(1H,s,$H_5$ of thiazole), 8.53(2H,d,$H_4$ and $H_6$ of pyrimidine)

IR $\nu_{max}^{KBr}$: 3450, 1750, 1660, 1560, 1770, 1380, 1270, 1220, 1110, 1045 $cm^{-1}$

E. Preparation of the titled compound (I)

The compound (I, R = H) (125 mg, 0.188 mmole) obtained by the method described in the part D in Example 3 was mixed with zinc powder (126 mg, 10.3 eq) in THF-1M $KH_2PO_4$ (8 ml - 2 ml) and stirred for 4.5 hours. Insoluble materials were filtered off and washed with EtOH. The combined filtrate and washings were condensed to a yellow powder which was then chromatographed on Wakogel C-200 (25 g, L/D = 68, EtOAc: EtOH : $H_2O$ = 15 : 3 : 2) to give the desired compound (I) (51 mg, 56 %) as off-white solids. The starting material (35 mg, 28 %) was recovered.

$R_f^{511}$ = 0.18, $R_f^{311}$ = 0.50

$^1H$ NMR(90MHz, $CD_3OD$): δ 2.0-2.8(2H,m,$C\underline{H}_2CH_2S$), 3.3(2H,m,$CH_2C\underline{H}_2S$), 4.29(1H,m,$H_4$), 4.84(1H,d, J=2.9Hz,$H_3$), 6.80(1H,s,$H_5$ of thiazole), 7.93(1H, t,$H_5$ of pyrimidine), 8.55(2H,d,J=5.1Hz, $H_4$ and $H_6$ of pyrimidine)

IR $\nu_{max}^{KBr}$: 3430, 1760, 1660, 1620 (sh.), 1570, 1550, 1530, 1380, 1270, 1240, 1050 $cm^{-1}$

UV $\lambda_{max}^{H_2O}$: 249 nm (ξ 21,800), 292 nm (ξ 7,970)

Example 4

Preparation of Potassium [3R,4R]-3-[2R-2-(4-hydroxyphenyl)-2-(4-ethyl-2,3-dioxo-1-piperadinecarboxamido)]-acetamido-4-[2-(2-pyrimidylthio)ethyl]-2-azetidinone-1-sulfonate (I)

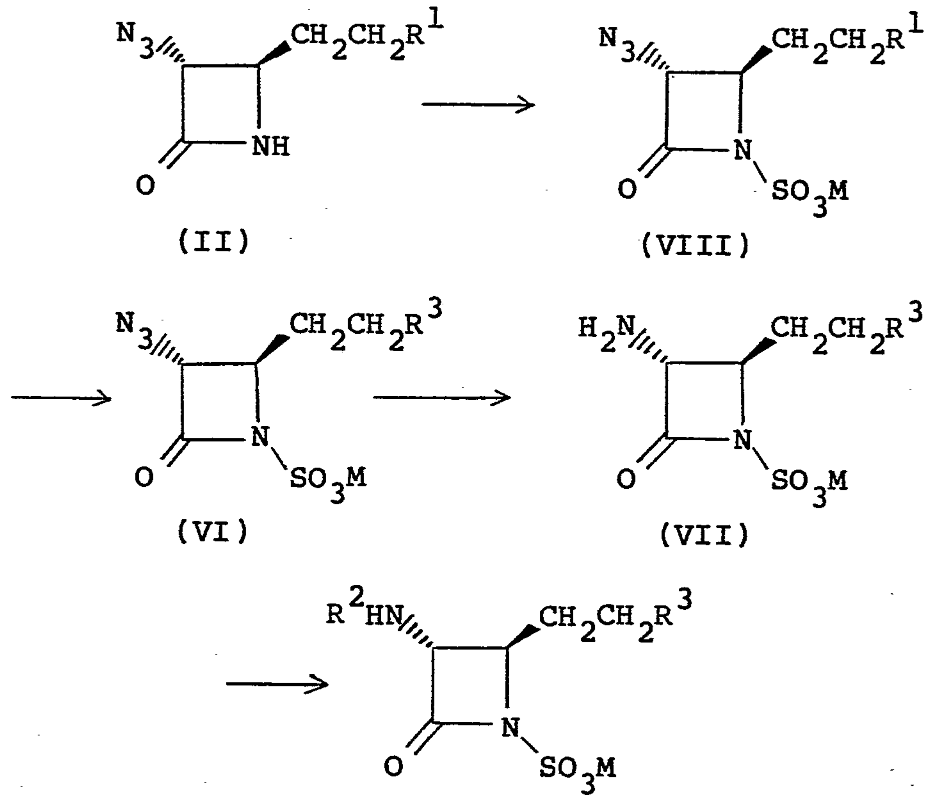

(II) (VIII) (VI) (VII) (I)

($R^1$ = $OSO_2Me$; $R^3$ = 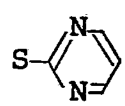; M = n-$Bu_4N$ or K;

$R^2$ = 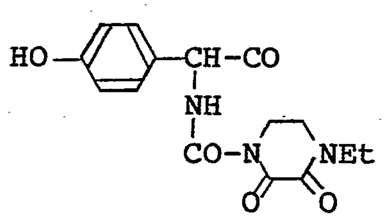

A. Preparation of tetra-n-butylammonium [3R,4R]-3-azido-4-(2-mesyloxy)ethyl-2-azetidinone-1-sulfonate (VIII)

To a solution of the compound (II) (3.5 g, 14.8 mmoles) in dry DMF (50 ml) was added the $SO_3$-pyridine complex (8.3 g, 3.5 eq) and the mixture was stirred at 60°C for 3 hours. The reaction mixture cooled to a room

temperature was poured into 1M $KH_2PO_4$ (100 ml) and the solution was adjusted to pH 6 with 2H NaOH. Neutral organic substances were removed by extracting with methylene chloride (100 ml) and tetra-n-butylammonium hydrogen sulfate (6 g, 1.19 eq) was added to the aqueous solution. The desired compound was then extracted with methylene chloride (300 ml + 150 ml), washed with saturated aq. NaCl (150 ml) and dried over anhydrous $MgSO_4$. Removal of the organic solvent under reduced pressure left a yellow syrup of the compound (VIII) (7.76 g, 94 %).

$R_f^{511}$ = 0.47, $[\alpha]_D^{25}$ + 74.5° (c 2.0, MeOH)

$^1H$ NMR(90MHz, $CDCl_3$): $\delta$ 1.0(12H,m,$CH_3$ of Bu), 1.5(16H,m,$CH_2$ of Bu), 2.5(2H,m,$C\underline{H}_2CH_2O$), 3.10(3H, s,$SO_2CH_3$), 3.2(8H,m,CH of Bu), 3.85(1H,m,$H_4$), 4.33(1H,d,J=2.3Hz,$H_3$), 4.50(2H,t,J=5.4Hz, $CH_2C\underline{H}_2O$)

IR $\nu_{max}^{neat}$: 2100 ($N_3$), 1760 ($\beta$-lactam), 1350, 1270, 1250, 1175, 1040 $cm^{-1}$

B. Preparation of tetra-n-butylammonium [3R,4R]-3-diazo-4-[2-(2-pyrimidylthio)ethyl]-2-azetidinone-1-sulfonate (VI)

By substituting the compound (VIII) prepared by the preceding process A for the compound (II) in the procedure of part A in Example 1, the desired compound (VI) (100 %) was obtained as a yellow syrup.

$R_f^{TE41}$ = 0.1, $[\alpha]_D^{25}$ + 41.5° (c 2.04, MeOH)

$^1H$ NMR(90MHz, $CDCl_3$): $\delta$ 3.90(1H,m,$H_4$), 4.37(1H,d,$H_3$)

IR $\nu_{max}^{neat}$: 3500, 2950, 2880, 2105, 1770, 1560, 1545, 1040 $cm^{-1}$

C. Preparation of tetra-n-butylammonium [3R,4R]-3-amino-4-[2-(2-pyrimidylthio)ethyl]-2-azetidinone-1-sulfonate

(VII)

By substituting the compound (VI) for the compound (IV) in the procedure of part B in Example 1, the compound (VII) (81 %) was prepared as a yellow syrup.

$R_f^{311} = 0.37$

D. Preparation of the compound (I)

By substituting (2R)-2-(4-hydroxyphenyl)-2-(4-ethyl-2,3-dioxo-1-piperadinecarboxamido)acetic acid for the (Z)-2-[2-(2,2,2-trichloroethoxycarbonyl)aminothiazol-4-yl]-2-methoxyiminoacetic acid in the procedure of the part C in Example 1, the tetra-n-butylammonium salt of the compound (I) (85 %) was obtained as a brown syrup.

$R_f^{511} = 0.21$, $R_f^{311} = 0.45$

$^1H$ NMR(90MHz, $CD_3OD$): $\delta$ 6.7, 7.2(2H,2H,d,d, phenylene), 7.03(1H,t,pyrimidine $H_5$), 8.49(2H,d, pyrimidine $H_4$ and $H_6$)

The syrup (460 mg, 0.668 mmole) was dissolved in 50 % aq. acetone and charged to a column of Dowex 50W X2 ($K^+$ form, 15 ml) which was then dveloped with 50 % aq. acetone. The fractions containing the potassium salt of the compound (I) were combined and acetone was evaporated off under reduced pressure. The resultant aqueous solution was charged to a column of Diaion HP-20 (17 ml) which was washed with deionized water (100 ml) and eluted with linear gradient method of deionized water and 50 % EtOH to give a pure potassium salt of the desired compound (I) (220 mg, 62 %).

$R_f^{311} = 0.35$

$^1H$ NMR(90MHz, $D_2O$): $\delta$ (DOH was adjusted to 4.7 ppm) 4.58(1H,d,$H_3$), 6.7-7.2(4H,d-d,phenylene), 7.1(1H, t,pyrimidine $H_5$), 8.45(2H,d,pyrimidine $H_4$ and $H_6$)

Example 5

Preparation of potassium (Z)-[3R,4R]-3-[2-(2-aminothiazol-4-yl)-2-methoxyimino]acetamido-4-[2-(2-pyridylthio)ethyl]-2-azetidinone-1-sulfonate (I).

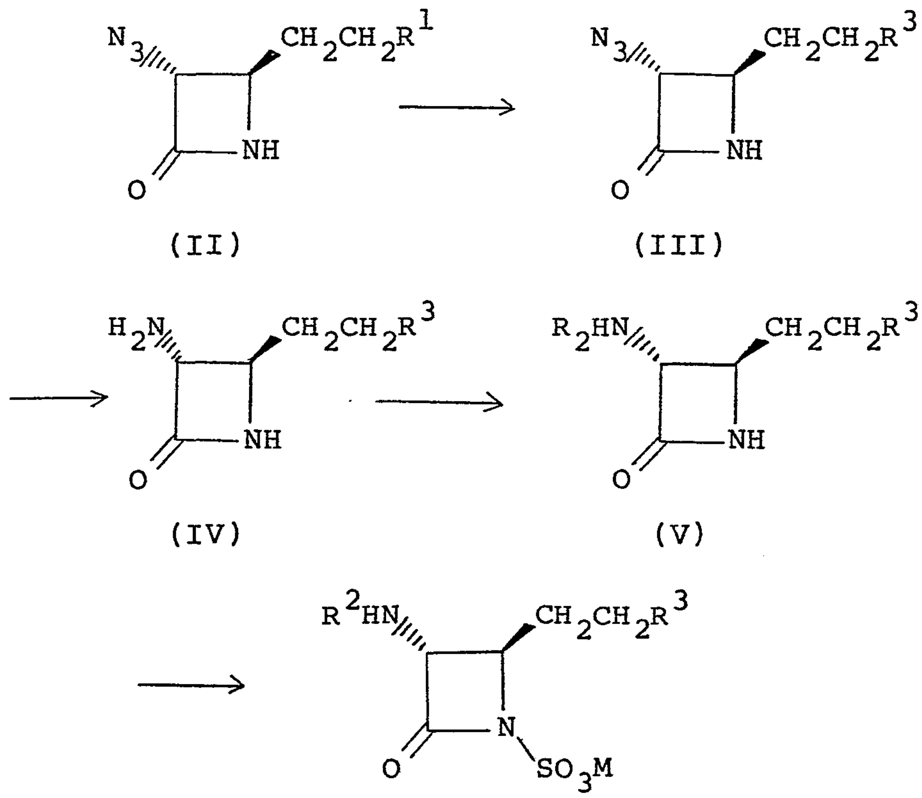

(II) (III)

(IV) (V)

(I)

[$R^1$ = $OSO_2Me$; $R^3$ = 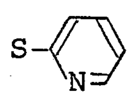; $R^2$ = 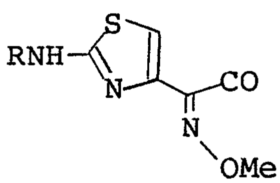

(R = H or 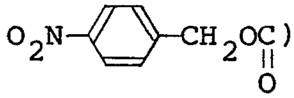)

A. Preparation of [3R,4R]-3-azido-4-[2-(2-pyridylthio)-ethyl]-2-azetidinone (III).

By substituting 2-mercaptopyridine for 2-mercapto-pyrimidine in the procedure of the part A in Example 1, the compound (III) (489 mg, 93 %) was obtained as a pale yellow syrup.

$R_f^{CA31} = 0.51$, $R_f^{TA91} = 0.13$, $[\alpha]_D^{25} + 157^o$ (c 2.22, MeOH)

$^1$H-NMR990MHz, $CDCl_3$) : $\delta$ 1.96-2.3(2H,m,$C\underline{H}_2CH_2S$), 3.0-3.5 (2H,m,$CH_2C\underline{H}_2S$), 3.53-3.8(1H,m,$H_4$), 4.3(1H,d,$H_3$), 6.8-7.63(4H,m,pyridine $H_3$,$H_4$,$H_5$ and β-lactam NH), 8.4(1H,d-d,pyridine $H_6$)

IR $\nu_{max}^{neat}$ : 3250, 2930, 2100, 1760, 1570, 1450, 1410, 1260, 1120, 760 $cm^{-1}$.

$n_D^{25}$ : 1.5923

B. Preparation of [3R,4R]-3-amino-[2-(2-pyridylthio)-ethyl]-2-azetidinone (IV).

By substituting the pyridylthio derivative (III) for the pyrimidylthio derivative (III) in the procedure of the part B in Example 1, the amine (IV) (250 mg, 58 %) was prepared as a pale yellow syrup.

$R_f^{TA11} = 0.8$

$^1$H NMR(90 MHz, $CDCl_3$) : $\delta$ 1.26(2H,d,J = 3Hz,$NH_2$), 1.8-2.35(2H,m,$C\underline{H}_2CH_2S$), 2.5-3.6(3H,m,$CH_2C\underline{H}_2S$,$H_4$), 3.8(1H,d-d,$H_3$), 6.7-7.65(4H,m,pyridine $H_3$,$H_4$,$H_5$ and β-lactam NH), 8.4(1H,d-d,pyridine $H_6$)

C. Preparation of (Z)-[3R,4R]-3-[2-(2-(4-nitrobenzyloxycarbonyl)aminothiazol-4-yl]-2-methoxyiminio]acetamido-4-[2-(2-pyridylthio)ethyl]-2-azetidinone (V)

By substituting (Z)-2-[2-(4-nitrobenzylcarbonyl)aminothiazol-4-yl]-2-methoxyimino-acetic acid for the corresponding 2-(2,2,2-trichloro-ethoxycarbonylamino) derivative in the procedure of the part C in Example 1, the compound (V) (250 mg, 38 %) was obtained.

$R_f^{TA12} = 0.33$

D. Preparation of the compound (I)

The mixture of the compound (V) and $SO_3$-pyridine complex (250 mg, 0.427 mmole and 238 mg, 3.5 eq, respectively) in dry DMF (5 ml) was stirred at 60$^o$C for 2 hours. The usual work up as described in the part D in Example 1 gave a pale yellow solid of potassium (Z)-[3R,4R]-3-[2-[2-(4-nitrobenzyloxycarbonyl)amino-

thiazol-4-yl]-2-methoxyimino acetamido-4-[2-(2-pyridylthio)ethyl]-2-azetidinone-1-sulfonate (246 mg, 84 %). The solid (240 mg) was dissolved in phosphate buffer (0.5 M, pH 6, 15 ml) and hydrogenated by using 10 % Pd/carbon (240 mg) as a catalyst at a room temperature for 6 hours. The mixture was then filtered through Hyflo Super-Cel and the cake was washed with fresh water. Neutral organic substances were extracted with ethyl acetate from the combined filtrate and washings and the aqueous solution was subjected to a column chromatography with Diaion HP-20 (50 ml, linear gradient elution of 0 - 30 % acetone in water) to give a pale yellow solid of the desired compound (I) (120 mg, 68 %).

$R_f^{411}$ = 0.18

IR $\nu_{max}^{KBr}$ : 3430, 1760, 1660, 1240, 1045 $cm^{-1}$.

<u>Example 6</u>

Preparation of Potassium (Z)-[3R,4R]-3-[2-(2-aminothiazol-4-yl)-2-methoxyimino]acetamido-4-[2-(2-benzothiazolthio)ethyl]-2-azetidinone-1-sulfonate (I).

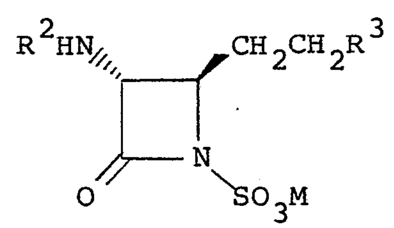

(I)

($R^2$ = 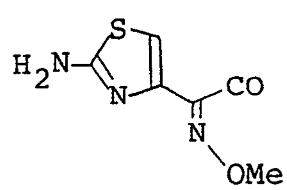 ; $R^3$ = 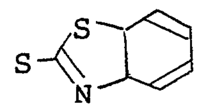 ; M = $K^+$)

By using 2-mercaptobenzothiazole instead of 2-mercapto-pyrimidine and reacting in the same manner as in Example 1, the compound (I) was prepared.

$R_f^{511}$ = 0.24

$^1H$ NMR(90MHz, $D_2O$) : $\delta$ (DOH was adjusted to 4.7 ppm) 3.9(3H,s,$OCH_3$), 6.63(1H,s,aminothiazole $H_5$), 7.03-7.66(4H,m,benzothiazole)

## Example 7

Preparation of Potassium (Z)-[3R,4R]-3-[2-(2-aminothiazol-4-yl)-2-methoxyimino]acetamido-4-ethyl-2-azetidinone-1-sulfonate (I)

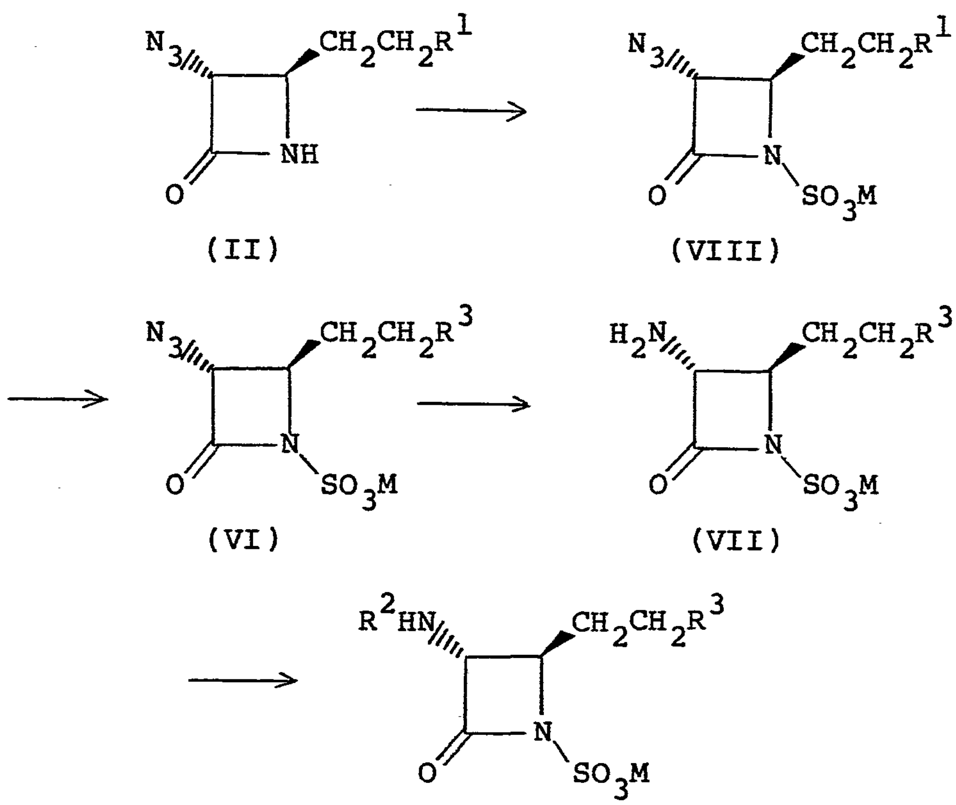

(II) (VIII) (VI) (VII) (I)

($R^1$ = $OSO_2Me$ or I; $R^2$ = RNH- 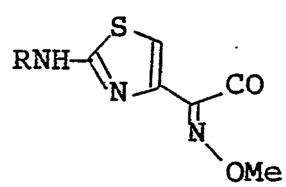 ; $R^3$ = H ; M = $K^+$)

A. Preparation of tetra-n-butylammonium [3R,4R]-3-azido-4-(2-iodoethyl)-2-azetidinone-1-sulfonate (VIII)

The iodide (II) (110 mg, 0.413 mmole), prepared by the same method as described in the part A of Example 2 except for using the (3R)-enantiomer of (II) ($R^1$ = $OSO_2Me$) instead of the (3S)-enantiomer, was mixed with $SO_3$-pyridine complex (180 mg, 2.7 eq) in dry DMF (1.5 ml) and warmed at 60°C for 5 hours. Working up in the same manner as in the procedure of the part A in Example 4 gave the compound (VIII) (M = $NBu_4$) as a colorless oil (218 mg, 90 %).

0108994

$R_f^{511} = 0.56$

$^1H$ NMR (90MHz, $CDCl_3$) : $\delta$ 1.0 (12H,t,$CH_2CH_2CH_2C\underline{H}_3$), 1.3-2.0 (16H,m,$CH_2C\underline{H}_2C\underline{H}_2CH_3$), 2.2-3.0 (2H,m,$C\underline{H}_2CH_2I$), 3.2-3.5 (10H,m,$C\underline{H}_2CH_2CH_2CH_3$, $CH_2C\underline{H}_2I$), 3.8 (1H,m,$H_4$), 4.23 (1H,d,J = 2.4Hz,$H_3$)

B. Preparation of the compound (I)

The compound (VIII) (218 mg, 0.371 mmole) was dissolved in a mixture of EtOH (1 ml), MeOH (3ml) and DMF (0.5 ml), and stirred at a room temperature after addition of 5 % Pd/C (29 mg, 2.7 wt % of Pd) and diethylamine (27 mg, 1 eq) under hydrogen atmosphere for 17 hours. The reaction mixture was then filtered through Hyflo Super-Cel, washed with fresh MeOH, and the combined filtrate and washings were condensed to a syrup. The syrup was dissolved in dry DMF (3 ml) and mixed with (Z)-2-(2-aminothiazol-4-yl)-2-methoxyiminoacetic acid (88 mg, 1.17 eq), 1-N-hydroxybenzotriazole monohydrate (66 mg, 1.16 eq) and DCC (112 mg, 1.46 eq), and the mixture was stirred at a room temperature for 20 hours. After the usual work up and column chromatography with Wakogel C-200 (9 g, toluene: acetone = 1 : 1 to 1 : 2), a glassy solid (37 mg) was obtained. The pure desired compound (I) (23 mg, 15 %) was isolated by column chromatography with Dowex 50W X2($K^+$ form, 10 ml).

$R_f^{511} = 0.18$, $R_f^{311} = 0.52$

$^1H$ NMR(90MHz, $D_2O$) : $\delta$ (DOH was adjusted to 4.7 ppm) 1.0(3H,t,J = 5.7Hz,$CH_2C\underline{H}_3$), 1.5(2H,m,$C\underline{H}_2CH_3$), 3.7(1H,m,$H_4$), 4.03(3H,s,$OCH_3$), 4.83(1H,d,J = 3.3Hz,$H_3$), 7.13(1H,s,aminothiazole $H_5$)

## Example 8

Preparation of Potassium [3R,4R]-3-[2-(2-aminothiazol-4-yl)-2-methoxyimino]acetamido-4-chloro-2-azetidinone-1-sulfonate (I)

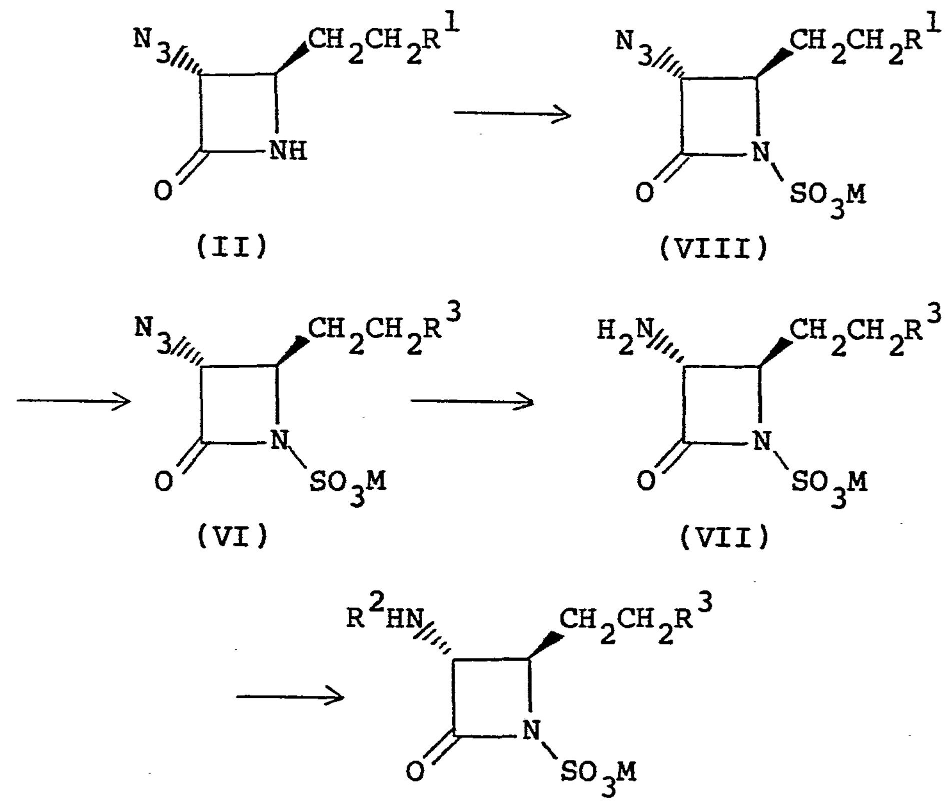

(II) (VIII) (VI) (VII)

(I)

($R^1$ = $OSO_2Me$ ; $R^2$ = 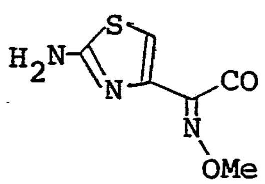 ; $R^3$ = Cl ; M = $K^+$)

A. Preparation of tetra-n-butylammonium [3R,4R]-3-azido-4-(2-chloethyl)-2-azetidinone-1-sulfonate (VI)

The compound (VIII) (606 mg, 1.09 mmole), prepared by the same procedure as that of the part A in Example 4, was mixed with dry LiCl (69.4 mg, 1.5 eq) in dry DMF (5 ml) and stirred at 60°C for 4 hours. The mixture was cooled and diluted with water (50 ml), and the product was extracted with ethyl acetate (50 ml X 3). The organic layer was washed with saturated aq. NaCl and dried over anhydrous $MgSO_4$. Evaporation of the solvent under reduced pressure gave a yellow syrup of the compound (VI) (458 mg, 85 %).

$R_f^{511} = 0.59$

$^1H$ NMR(90MHz, $CCl_4$): $\delta$ 2.1-2.9(2H,m,$C\underline{H}_2CH_2Cl$), 3.80(2H,m,$CH_2C\underline{H}_2Cl$), 3.93(1H,d-d,$H_4$), 4.40(1H,d, J = 2.4Hz,$H_3$)

IR $\nu_{max}^{neat}$ : 3500, 2950, 2880, 2100, 1750, 1260, 1040 $cm^{-1}$.

B. Preparation of tetra-n-ammonium [3R,4R]-3-amino-4-(2-chloroethyl)-2-azetidinone-1-sulfonate (VII)

To the solution of the compound (VI) (385 mg, 0.776 mmole) in EtOH (10 ml) was added 5 % Pd/C (138 mg, 1.8 wt % of Pd) and the mixture was stirred at a room temperature under hydrogen atmosphere for 4 hours. TLC showed only one spot of the compound (VII). The usual work up gave a pale yellow oil of the compound (VII) (365 mg, 99.5 %).

$R_f^{511}$ = 0.37

$^1H$ NMR(90MHz, $CDCl_3$ : $CD_3OD$ = 1 : 1) : $\delta$ 2.1-2.9(2H, m,$C\underline{H}_2CH_2Cl$), 3.81(2H,m,$CH_2C\underline{H}_2Cl$), 3.89(2H,d-m,J = 2.2Hz,$H_3$ and $H_4$)

IR $\nu_{max}^{neat}$ : 2100($N_3$),1750($\beta$-lactam) $cm^{-1}$.

C. Preparation of the compound (I)

The compound (VII) (363 mg, 0.772 mmole) was acylatd with (Z)-2-(2-aminothiazol-4-yl)-2-methoxyimino-acetic acid (1.19 eq) in the same procedure as that of part C in Example 2, and a crude tetra-n-butyl-ammonium salt of (I) was converted to a potassium salt by using Dowex 50W X2 column ($K^+$ form, 21 ml) and finally purified by Diaion HP-20 (25 ml, linear gradient elution of 0 - 50 % EtOH) to give off-white powder of the compound (I) (197 mg, 57 %).

$R_f^{411}$ = 0.31, $R_f^{311}$ = 0.52

$^1H$ NMR(90MHz, $D_2O$) : $\delta$ (DOH was adjusted to 4.7 ppm) 2.0-2.9(2H,m,$C\underline{H}_2CH_2Cl$), 3.82(2H,t,$CH_2C\underline{H}_2Cl$), 3.97(3H,s,$OCH_3$), 4.4(1H,m,$H_4$), 4.81(1H,d,J = 2.9Hz,$H_3$) 6.90(1H,s,aminothiazole $H_5$)

Example 9

Preparation of Potassium (Z)-[3R,4R]-3-[2-(2-aminothiazol-4-yl)-2-methoxyimino]acetamido-4-(2-thiocarbamoylthio)ethyl-2-azetidinone-1-sulfonate (I).

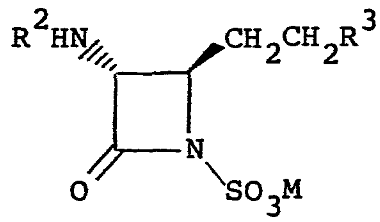

(I)

($R^2$ = 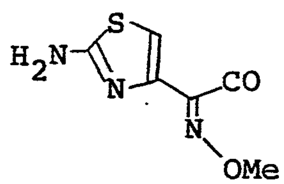 ; $R^3$ = 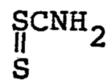 ; $M = K^+$)

A mixture of the chloride (I) (R=Cl, 190 mg, 0.2 mmole), prepared in the same manner as in Example 8, and ammonium dithiocarbamate (90 mg, 0.8 mmole) in dimethyl sulfoxide (DMSO, 2 ml) was stirred at a room temperature for 2 hours. To the mixture was added the phosphate buffer (0.2 M, pH 6.8, 15 ml) and saturated aq. NaCl (5 ml), and the resultant solution was charged to a column of Diaion HP-20 (21 ml) which was washed with deionized water (100 ml) and eluted with 40 % EtOH. Lyophillization of the fractions having bioactivity against E. coli gave off-white amorphous powder of the desired compound (I) (55 mg, 50 %).

$R_f^{311} = 0.50$

$^1H$ NMR(90MHz, $D_2O$) : $\delta$ (DOH was adjusted to 4.7 ppm) 2.0-2.8(2H,m,$C\underline{H}_2CH_2S$), 3.32(2H,t,$CH_2C\underline{H}_2S$), 3.97(3H,s,$OCH_3$), 4.77(1H,d-d,$H_4$), 5.03(1H,d,$H_3$), 6.87(1H,s,aminothiazole $H_5$)

IR $\nu_{max}^{KBr}$ : 3450, 1760, 1660, 1620, 1535, 1380, 1270, 1240, 1035 $cm^{-1}$.

Example 10

Preparation of [3R,4R]-3-azido-4-(2-chloroethyl)-2-azetidinone (III)

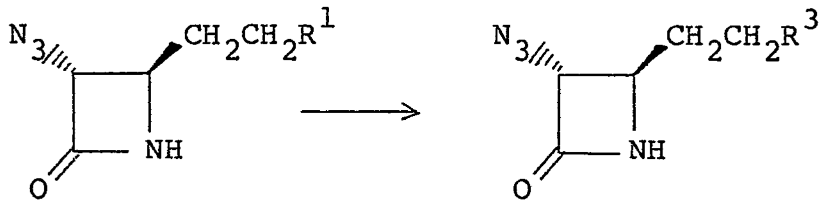

(II) (III)

($R^1$ = OTs ; $R^3$ = Cl)

By substituting the tosylate for the mesylate in the procedure of the part A in Example 8, the chloride (III) was obtained.

$R_f^{TA91}$ = 0.23, $[\alpha]_D^{25}$ + 145.7° (c 1.9, MeOH)

$^1H$ NMR(90MHz, $CDCl_3$) : $\delta$ 1.95-2.34(2H,q,$C\underline{H}_2CH_2Cl$), 3.65(2H,t,J = 6Hz,$CH_2C\underline{H}_2Cl$), 3.56-3.84(1H,m,$H_4$), 4.38(1H,d,J = 2Hz,$H_3$), 6.9(1H,b,NH)

## Example 11

Preparation of (Z)-[3S,4R]-3-[2-[2-(2,2,2-trichloroethoxycarbonyl)aminothiazol-4-yl]-2-[(1-carboxy-1-methyl-ethoxyimino]acetamido-4-[2-(2-pyrimidylthio)ethyl]-2-azetidinone (V)

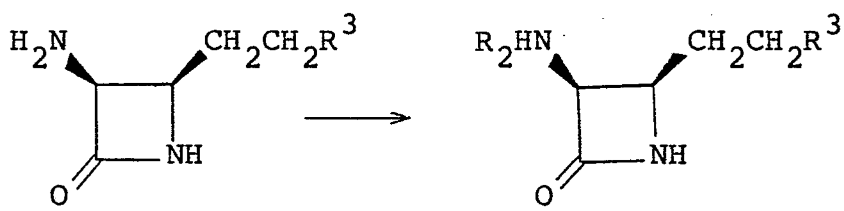

(IV) (V)

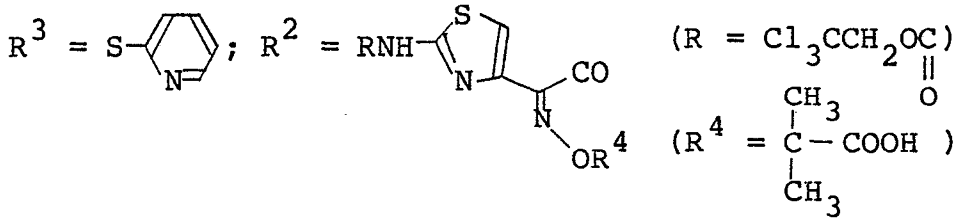

The activated ester of (Z)-2-[2-(2-trichloroethoxycarbonyl)aminothiazol-4-yl]-2-[1-(4-nitrobenzyloxy)carbonyl-1-methyl]ethoxyimino-acetic acid was prepared by reacting the acid with 1-N-mesyloxybenzotriazole in dry freshly distilled THF in the

presence of triethylamine at a room temperature for an hour. A solution of the amine (IV), which was obtained by the procedure described in the part A and B in Example 1, in dry freshly distilled THF was then added to the reaction mixture and stirred for 5 hours at a room temperature. Treating the mixture in the same manner as in the procedure of the part C in Example 1, the compound (V) wherein $R^4$ is [1-(4-nitrobenzyloxy)carbonyl-1-methyl]ethyl group was isolated.

$R_f^{511}$ = 0.76 (amine (IV) = 0.31 ; acid = 0.33)

$^1H$ NMR(90MHz,$CDCl_3$) : $\delta$ 1.62(6H,s,$CH_3$), 2.0(2H,m,$C\underline{H}_2CH_2S$), 3.2(2H,m,$CH_2C\underline{H}_2S$), 4.76(1H,m,$H_4$), 4.86(2H,s,$Cl_3CCH_2O$), 5.22(2H,s,benzyl), 5.35(1H,d,$H_3$), 6.91(1H,t,pyrimidine $H_5$), 7.25(1H,s,thiazole $H_5$), 7.43(2H,d,phenylene $H_3$,$H_5$), 8.07 (2H,d,phenylene $H_2$,$H_6$), 8.44(2H,d,pyrimidine $H_4$,$H_6$)

The desired compound (V) was obtained by hydrogenative deprotection of 4-nitrobenzyl group using 10 % Pd/C catalyst in a mixture of ethyl acetate and ethanol as a solvent.

$R_f^{511}$ = 0.18

Example 12

Preparation of dipotassium (Z)-[3S,4R]-3-[2-(2-aminothiazol-4-yl)-2-[1-carboxy-1-methyl)ethoxyimino]-acetamido-4-[2-(2-pyrimidylthio)ethyl]-2-azetidinone-1-sulfonate (I)

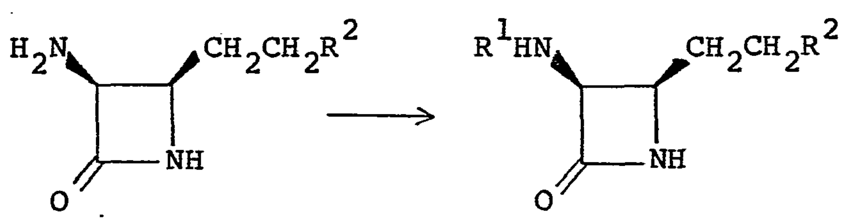

(III) (IV)

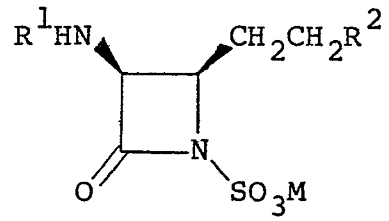

(I)

$R^2$ = 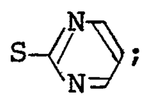; $R^1$ = 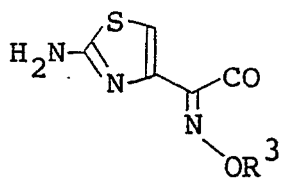 , ($R^3$ = 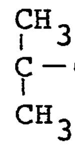COOK);M = K

The compound (IV) was prepard by activated ester method as described in Example 11, using (Z)-2-[2-aminothiazol-4-yl-2-[1-(4-nitrobenzyloxy)carbonyl-1-methyl]ethoxyimino]-acetic acid, from the compound (III) in dry DMF at a room temperature. The compound (IV) was extracted with EtOAc from a diluted aqueous reaction mixture with 0.5 M phosphate buffer (pH6.8), and without purification treated with the complex, pyridine-$SO_3$ and worked up as described in Example 1. The desired product (I) was isolated by gel absorption column chromatography using Diaion HP-20, developing with a linear gradient solution of 0 to 30 % acetone in water.

$R_f^{311}$ = 0.27

$^1H$ NMR (90MHz,$D_2O$) : $\delta$ (DOH was adjusted to 4.7 ppm) 1.40 (6H,$CH_3$), 2.28(2H,m,$CH_2\underline{CH_2}S$), 3.25 (2H,m, $\underline{CH_2}CH_2S$), 4.50 (1H,m,$H_4$), 5.41 (1H,d,$H_3$), 6.84 (1H,s,thiazole $H_5$), 7.15 (1H,t,pyrimidine,$H_5$), 8.51 (2H,d,pyrimidine,$H_4$,$H_6$)

## Example 13

Preparation of the compound (III)

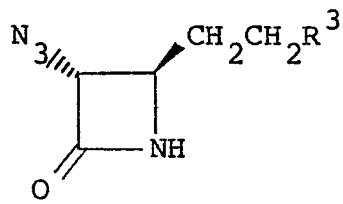

(III)

($R^3$ = 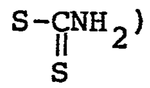

To a solution of chloride (III) (100 mg, 0.308 mmole) obtained in Example 10 in DMSO (2 ml) was added ammonium dithiocarbamate (70 mg, 2 eq), and the reaction mixture was subjected to reaction at a room temepratur for one hour. After removing DMSO by lyophillization, yellow syrup of the desired compound (II) (80 mg, 81 %) was obtained by dry column chromatography of Wako gel C-200 (7 g) (EtOAc : EtOH = 1 : 1).

$R_f^{511}$ = 0.56

$^1H$ NMR (90 MHz,$CDCl_3$ : $CD_3OD$ = 1 : 1) : $\delta$ 2.10 (2H, q,$C\underline{H}_2$,$CH_2Cl$), 3.30 (2H,t,$CH_2C\underline{H}_2Cl$), 3.60 (1H,t-d, $H_4$), 4.35 (1H,d,J=2.2 Hz)

## Example 14

Preparation of the compound (III)

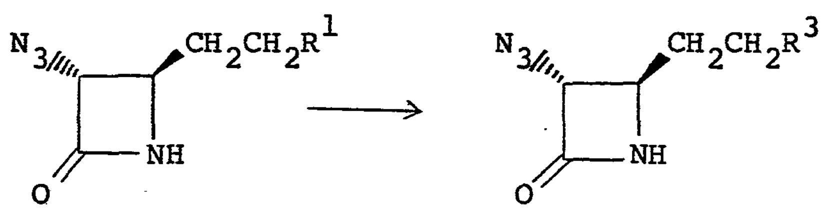

(II) (III)

$R^1$ = $OSO_2Me$ or I; $R^3$ = 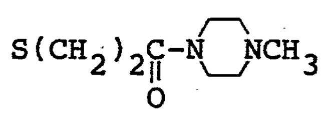

To the compound (II) (240 mg, 1.02 mmoles), prepared in the same manner as described in the part A of Example 2 except for using the (3R)-enatiomer of (II) ($R^1$ = $OSO_2Me$) instead of the (3S)-enatiomer, were added dry DMF (6 ml) and potassium salt of N-methyl-N-(2(S)-2-methyl-3-mercaptpropanoyl)piperazine (0.25 g, 1.04 mmoles), and the reaction mixture was stirred at $50^o$ to $60^oC$ for about 16 hours under nitrogen atmosphere. After

removal of DMF under reduced pressure, the oily residue was purified by chromatography on silica gel (solvent system: TA12). The fractions having $R_f^{TA12}$ = 0.18 were combined to give a colorless oil of the compound (III) (160 mg, 46 %).

$^1$H NMR (90MHz, $CDCl_3$) 1.16 (3H,d,$CHC\underline{H}_3$), 1.83-2.20 (2H,m,$SCH_2C\underline{H}_2$), 2.3 (3H,s,$NCH_3$), 2.3-3.2 (10H,m, $NCH_2C\underline{H}_2NCH_3$,$C\underline{H}CH_3$,$CH_2SC\underline{H}_2$,$H_4$), 3.46-3.76 (4H,m, $NC\underline{H}_2CH_2NCH_3$), 4.25 (1H,d,$H_3$), 7.58 (1H,broad S, N$\underline{H}$)

## Example 15

Preparation of the compound (VI)

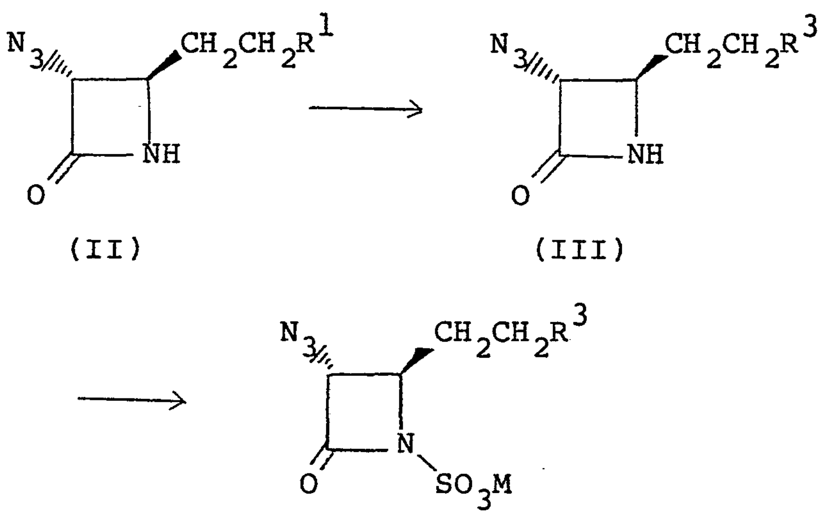

(II) (III)

(VI)

($R^1$ = $OSO_2Me$ or I; $R^3$ = 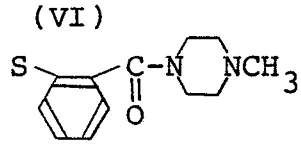 ; M = $NBu_4$)

A. Preparation of compound (III)

To thiosalicylic acid (760 mg, 5 mmoles) dissolved in dry THF (7 ml) was added N-methyl-piperazine (1.02 g, 10 mmoles) in THF (7 ml). To the resulting mixture were further added 4-dimethylaminopyridine (60 mg, 0.5 mmole), DCC (1.05 g, 5.1 mmoles) and THF (5 ml), and the reaction mixture was stirred at a room temperature for 5.5 hours under nitrogen atmosphere. After filtration of the insoluble solid, the filtrate was washed with THF and acetone, and the solvent was

evaporated. After filtration of the insoluble solids by addition of methylene chloride, the filtrate was washed with methylene chloride to give a yellow oily material (930 mg). To the resultant oily material were added the compound (II) (260 mg, 1.11 mmoles) and dry DMF (10 ml), and the reaction mixture was stirred at 60° to 65°C for about 16 hours under nitrogen atmosphere. After removal of DMF under reduced pressure, the reaction mixture was extracted with water, sodium bicarbonate and methylene chloride. The organic layer was dried over anhydrous $MgSO_4$ and filtered, and removal of the solvent left a mixture of solid and oil (930 mg) which was purified by chromatography on silica gel to give a pale yellow oily material (160 mg). The resultant oily material was dissolved in methylene chloride and extracted with diluted HCl. The aqueous layer was adjusted to pH 7.8 with aq. $NaH_2CO_3$, extracted with methylene chloride and dried over anhydrous $MgSO_4$. After filtration and evaporation of the solvent, very pale yellow oil of the compound (III) (140 mg, 33 %) was obtained.

$R_f^{311} = 0.42$

B. Preparation of the compound (VI).

The compound (III) obtained in the part A of Example 15 was sulfonated in the procedure of the part B in Example 2 to give the desired compound (VI) as a yellow syrup (180 mg).

$R_f^{311} = 0.45$

$^1$H NMR (90MHz,$CDCl_3$): $\delta$ 0.97 (12H,t,Bu), 1.16-1.83 (16H,m,Bu), 1.86-2.2 (2H,m,$C\underline{H}_2CH_2S$), 2.33 (3H,S, N-$CH_3$), 2.2-2.5 (4H,m,N-$CH_2C\underline{H}_2$-N-$CH_3$), 3.06-3.36 (8H,m,Bu), 3.56 (2H,t,S-$C\underline{H}_2$-$CH_2$), 3.66-3.93 (5H, m,$H_4$,$NCH_2$-), 4.8 (1H,d,$H_3$), 7.06-7.56 (4H,m, aromatic H)

WHAT WE CLAIM IS:

1. A process for the preparation of an optically active (4R)-substituted monocyclic β-lactam compound represented by the formula (I):

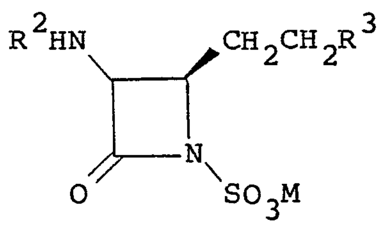

(I)

wherein $R^2$ ia an acyl group: $R^3$ is a hydrogen atom, hydroxyl, an alkoxy, azido, amino, formimidoylamino, cyano, thiocyanato, a substituted thio, dithiocarbamoyl, nitro group, or a halogen atom; M is hydrogen atom or a cation, which comprises subjecting a starting optically active compound represented by the formula (II):

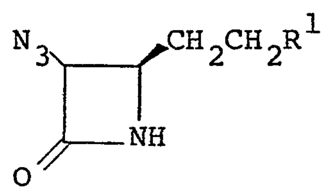

(II)

wherein $R^1$ is alkylsulfonyloxy, aralkylsulfonyloxy, arylsulfonyloxy group or a halogen atom,
to reaction comprising the steps of

(a) conversion of the azido group at C-3 position into amino group with a reducing reagent;
(b) acylation of the amino group;
(c) substitution of the $R^1$ group at C-4 position for a nucleophile and
(d) sulfonation of the N-1 position at a temperature of about $20^oC$ to about $70^oC$.

2. The process of Claim 1, wherein $R^1$ is mesyloxy or tosyloxy group.

3. The process of Claim 1, wherein $R^2$ is an

0108994

acyl group represented by the formula (III):

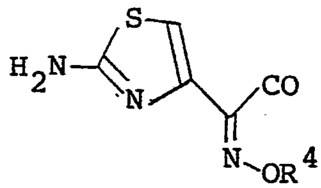

(III)

wherein $R^4$ is methyl, $CH_2COOR^5$ or $-C(CH_3)_2-COOR^5$ ($R^5$ is hydrogen atom, a cation or an ester group).

4. The process of Claim 1, wherein $R^3$ is 2-pyrimidylthio or 2-pyridylthio group.

European Patent Office

# EUROPEAN SEARCH REPORT

0108994 Application number

EP 83 11 0884

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 053 816 (TAKEDA) * Claims * | 1,3 | C 07 D 417/14<br>C 07 D 403/14<br>C 07 D 417/12<br>C 07 D 205/08<br>A 61 K 31/505<br>A 61 K 31/44<br>A 61 K 31/425<br>A 61 K 31/395//<br>C 07 D 403/12<br>C 07 D 401/12 |
| Y | EP-A-0 048 953 (SQUIBB) * Claims * | 1,3 | |
| D,A | THE JOURNAL OF ORGANIC CHEMISTRY, vol. 47, no. 1, January 1, 1982, pages 176-178 D.M. FLOYD et al.: "Monobactams. Stereospecific synthesis of (S)-3-amino-2-oxoazetidine-1-sulfonic acids" * Whole article * | 1 | |
| P,Y | FR-A-2 515 182 (ROUSSEL-UCLAF) * Claims * | 1,3 | |
| | | | TECHNICAL FIELDS SEARCHED (Int. Cl. 3) |
| | | | C 07 D 417/00<br>C 07 D 403/00<br>C 07 D 205/00<br>A 61 K 31/00 |

The present search report has been drawn up for all claims

| Place of search | Date of completion of the search | Examiner |
|---|---|---|
| THE HAGUE | 01-02-1984 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82